(19) Europäisches Patentamt
European Patent Office
Office européen des brevets

(11) EP 0 635 008 B1

(12) **EUROPÄISCHE PATENTSCHRIFT**

(45) Veröffentlichungstag und Bekanntmachung des Hinweises auf die Patenterteilung:
**04.09.2002 Patentblatt 2002/36**

(21) Anmeldenummer: **94904943.1**

(22) Anmeldetag: **09.02.1994**

(51) Int Cl.[7]: **C07D 295/18**, C07D 295/22, C07D 311/70, A61K 31/495

(86) Internationale Anmeldenummer:
**PCT/CH94/00026**

(87) Internationale Veröffentlichungsnummer:
**WO 94/018185 (18.08.1994 Gazette 1994/19)**

(54) **PIPERAZIDE VON SUBSTITUIERTEN PHENYLALANIN-DERIVATIVEN ALS THROMBIN INHIBITOREN**

PIPERAZIDES OF SUBSTITUTED PHENYLALANINE DERIVATES AS THROMBIN INHIBITORS

PIPERAZIDES DE DERIVES SUBSTITUES DE PHENYLALANINE UTILISES COMME INHIBITEURS DE LA THROMBINE

(84) Benannte Vertragsstaaten:
**AT BE CH DE DK ES FR GB IT LI LU NL SE**

(30) Priorität: **10.02.1993 CH 40593**
**20.09.1993 CH 282793**

(43) Veröffentlichungstag der Anmeldung:
**25.01.1995 Patentblatt 1995/04**

(73) Patentinhaber: **Pentapharm A.G.**
**CH-4052 Basel (CH)**

(72) Erfinder:
- **STÜRZEBECHER, Jörg**
  **D-99094 Erfurt-Rhoda (DE)**
- **VIEWEG, Helmut**
  **D-79618 Rheinfelden (DE)**
- **WIKSTROEM, Peter**
  **CH-4104 Oberwil (CH)**
- **ADLER, Christoph**
  **CH-4153 Reinach (CH)**

(74) Vertreter: **Braun, André, jr. et al**
**BRAUN & PARTNER**
**Patent-, Marken-, Rechtsanwälte**
**Reussstrasse 22**
**4054 Basel (CH)**

(56) Entgegenhaltungen:
**EP-A- 0 097 630**
**WO-A-92/08709**
**GB-A- 2 007 663**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 635 008 B1

Printed by Jouve, 75001 PARIS (FR)

## Beschreibung

Die vorliegende Erfindung betrifft neue Proteinasen-Inhibitoren, die als Grundstruktur Phenylalanin enthalten, wobei der aromatische Rest in meta-Stellung eine basische Gruppe besitzt und die α-Aminogruppe mit unterschiedlichen Resten sulfonyliert ist. Durch C-terminale Einführung verschiedenartig N-substituierter Piperazin-Reste wurden hochwirksame Inhibitoren mit verbesserter Bioverfügbarkeit gefunden.

Proteinase-Inhibitoren sind potentielle Arzneimittel, die zur Steuerung physiologischer Prozesse, welche durch Proteinasen ausgelöst und unterhalten werden, verwendet werden können. Für zahlreiche endogene bzw. natürlich vorkommende Hemmstoffe ist gezeigt worden, dass sie in vivo die Aktivität von Proteinasen beeinflussen und hyperproteolytische Zustände dämpfen können [Siehe Hörl, W.H. In: Design of Enzyme Inhibitors as Drugs, S. 573-581, (Sandler, M. and Smith, H.J., Eds.) Oxford, New York, Tokyo: Oxford University Press, 1989]. Der therapeutische Einsatz dieser relativ hochmolekularen Hemmstoffe ist allerdings wegen ihrer besonderen Proteinstruktur begrenzt. Da diese Hemmstoffe einerseits nach oraler Verabreichung im Darm nicht resorbierbar sind und andererseits eine antigene Aktivität ausüben, wurde nach synthetischen kleinmolekularen Enzym-Inhibitoren Ausschau gehalten.

Die vier Klassen von Enzymen, die für Proteinasen-abhängige Prozesse verantwortlich sind, umfassen die Serin-, Thiol-, Metallo- und Aspartat-Proteinasen. Serin-Proteinasen sind proteolytische Enzyme, die einen reaktiven Serin-Rest im aktiven Zentrum besitzen. Zur Trypsin-Familie der Serin-Proteinasen gehören Enzyme, die wie Trypsin als solches C-terminale Peptidbindungen der basischen Aminosäuren Arginin und Lysin spalten. In dieser Gruppe sind diejenigen Enzyme von besonderer physiologischer Bedeutung, welche im Blut Gerinnung und Fibrinolyse auslösen, Kinin freisetzen und die Komplement-Aktivierung bewirken oder solche, die selber Komponenten der genannten Enzymsysteme sind.

Die Blutgerinnung wird über zwei unterschiedliche Wege durch Zymogen-Aktivierung ausgelöst. Der erste, endogene Weg, führt über eine durch Blutkomponenten vermittelte Reaktionskette zur Blutgerinnung. Der zweite, exogene Weg, führt über eine kürzere, auf einer Wechselwirkung zwischen Blut- und Gewebekomponenten beruhenden Reaktionskette zur Gerinnung. Beide Wege bewirken die Aktivierung des Zymogens Faktor X zur Serin-Proteinase Faktor $X_a$, welche ihrerseits die Aktivierung des Prothrombins zur Fibrinogen-koagulierender Serin-Proteinase Thrombin katalysiert. Als gemeinsames Produkt sowohl des endogenen als auch des exogenen Aktivierungsablaufs wurde Faktor $X_a$ zunächst als ein bevorzugtes Zielenzym für hemmende Eingriffe in den Blutgerinnungsvorgang angesehen (Tidwell, R. R. et al., Thromb. Res. 19, 339-349, 1980). In jüngster Zeit wurde aber nachgewiesen, dass synthetische Inhibitoren des Faktors $X_a$ in vitro und in vivo nicht gerinnungshemmend (Stürzebecher, J. et al., Thromb. Res. 54, 245-252, 1989) und antithrombotisch wirksam sind (Hauptmann, J. et al., Thromb. Haemostas. 63, 220-223, 1990). Aus diesem Grund konzentriert sich die Entwicklung von antikoagulativ wirkenden Hemmstoffen auf die Auffindung von Inhibitoren des Thrombins.

Zur Entwicklung von synthetischen Inhibitoren für Thrombin sind Derivate des Benzamidins vielfach untersucht worden (Stürzebecher, J. et al., Acta Biol. Med. Germ. 35, 1665-1676, 1976). Dabei haben sich Aminosäurederivate mit Benzamidin-Struktur und para-ständiger Amidinogruppe als günstige Grundstrukturen für die Entwicklung von wirksamen Hemmstoffen erwiesen. So ist das Aminosäure-Derivat Nα-(2-Naphthylsulfonyl)-glycyl-4-amidinophenylalanin-piperidid (NAPAP) der bisher stärkste, beschriebene Thrombin-Hemmstoff ($K_i = 6 \times 10^{-9}$ Mol/Liter) vom Benzamidintyp (Stürzebecher, J. et al., Thromb. Res. 29, 635-642, 1983).

Es sind noch weitere Typen von Inhibitoren bekannt, die Thrombin ebenfalls wirksam hemmen: Eine erste Gruppe beinhaltet Peptidyl-arginin-chlormethylketone, z.B. H-D-Phe-Pro-Arg-$CH_2Cl$ (Kettner, C. et al., Thromb. Res. 14, 969-973, 1979). Eine zweite Gruppe beinhaltet Peptidylargininaldehyde, z.B. Boc-D-Phe-Pro-Arg-H und H-D-Phe-Pro-Arg-H (Bajusz, S., Int. J. Peptide Protein Res. 12, 217-221, 1978).

Diese Inhibitoren, die Trypsin und Thrombin mit vergleichbarer Affinität hemmen, sind jedoch schwierig zu synthetisieren, instabil und könnten wegen ihrer grossen Reaktionsfähigkeit unerwünschte Nebenreaktionen verursachen. Thrombin, aber auch Trypsin, wird in einer zeitabhängigen Reaktion ebenfalls durch das Boronsäurederivat Boc-D-Phe-Pro-Boro-Arg-$C_{10}H_{16}$ (s. Europ. Patentanmeldung No. 0 293 881) gehemmt. Dagegen hat der selektive Thrombininhibitor (2R,4R)-4-Methyl-1-[Na-(3-methyl-1,2,3,5-tetrahydro-8-chinolinsulfonyl)-L-arginin]-2-pipecolincarbonsäure praktisch keine Trypsin-hemmende Aktivität (Kikumoto, R. et al., Biochemistry 23, 85-90, 1984).

Alle bisher geprüften Benzamidinderivate besitzen für eine therapeutische Anwendung ungünstige pharmakodynamische und pharmakokinetische Eigenschaften. Sie werden bei oraler Applikation nicht im Darm resorbiert, werden schnell aus der Zirkulation eliminiert und ihre Toxizität ist relativ hoch. Das gilt sowohl für die Amide des N-α-arylsulfonylierten (Markwardt, F. et al., Thromb. Res. 17, 425-431, 1980), als auch für Amide des N-α-aryl-sulfonylaminoacylierten 4-Amidinophenylalanins (s. Patentanmeldung No. DD-A-235 866). Verantwortlich für die unzureichenden pharmakologischen Eigenschaften ist die stark basische Amidinofunktion (Kaiser, B. et al., Pharmazie 42, 119-121, 1987). Versuche, die stark basische Amidinofunktion in hochwirksamen Inhibitoren durch schwächer basische Gruppen zu ersetzen, hatten zunächst wenig Erfolg, da sie einen bedeutenden Verlust an Wirkungsstärke zur Folge hatten (Stürzebecher, J. et al., Pharmazie 43, 782-783, 1988). Auch die Einführung einer Carboxylgruppe in den Inhibitor zur

Verminderung der Basizität der Amidinofunktion führte zum Abfall der Inhibitoraktivität. So sind Derivate des 4-Amidinophenylalanins, die C-terminal eine Aminosäure mit freier Carboxylgruppe besitzen, inhibitorisch völlig wirkungslos (Wagner, G. et al., Pharmazie 39, 16-18, 1984; Vieweg, H. et al., Pharmazie 39, 82-86, 1984).

Auch die Abwandlung von NAPAP durch Einführung eines Substituenten am α-Stickstoff, die zu einer geringen Erhöhung der Antithrombinaktivität führte (s. Europ. Patentanmeldung No. FR-A-2 593 812) erbrachte keine Verbesserung der pharmakologischen Eigenschaften (Cadroy, Y. et al., Thromb. Haemostas. 58, 764-767, 1987).

Ausgehend von Nα-substituiertem 3-Amidinophenylalanin wurde die Entwicklung selektiver Hemmstoffe des Thrombins weitergeführt und gefunden, dass sowohl Amide vom Typ des Nα-2-naphthylsulfonylierten 3-Amidinophenylalanins mit einer Carboxylgruppe im Amidteil als auch solche Derivate, bei denen die Amidinofunktion durch eine andere basische Gruppe ausgetauscht war, verbesserte pharmakologische Eigenschaften aufwiesen. Insbesondere war erstmalig bei Benzamidinderivaten eine gewisse Resorption nach oraler Applikation gefunden worden (PCT/CH 9100235).

Diese Stoffklasse wurde nun weiterentwickelt. So zeigte sich beispielsweise bei der Einführung von neuen Substituenten am N-4-Atom von N-α-sulfonylierten 3-Amidinophenylalanin-piperaziden, insbesondere bei der Einführung von Acyl-(-CO-X), Sulfonyl ($-SO_2-Y$), Carbamoyl- (-CO-NR'R") oder funktionalisierten Alkyl-Resten, wobei X, Y sowie R',R" im günstigen Falle Methylgruppen darstellen und der funktionalisierte Alkylrest ($C_1$-$C_3$) eine OH-Gruppe trägt, dass die inhibitorische Wirksamkeit gegenüber Thrombin erheblich gesteigert werden konnte und überraschenderweise eine ausserordentliche Erhöhung der Resorptionsfähigkeit festzustellen war. Das war insbesondere nach rektaler aber auch nach intraduodenaler Applikation der Derivate, die sowohl in der Salzform als auch als freie Basen angewendet wurden, zu verzeichnen.

Dabei wurden nicht nur die razemischen Gemische dargestellt, sondern auch die reinen optischen Antipoden. In diesem Rahmen wurde beispielsweise Nα-(2-Naphthylsulfonyl)-3-amidino-(L)-phenylalanin-4-acetylpiperazid hergestellt. Es wurde festgestellt, dass diese Verbindung nicht nur ein starker Thrombinhemmstoff ist und die Gerinnung überaus wirksam beeinflusst, sondern überraschenderweise verbesserte pharmakokinetische Eigenschaften besitzt. Sie wird insbesondere nach rektaler Verabreichung an Ratten durch den Darm resorbiert und ist im Blut über einen längeren Zeitraum in blutgerinnungshemmender und antithrombotisch wirksamer Konzentration verfügbar. Dies trifft auch für Verbindungen mit anderen N-terminalen Schutzgruppen zu, die als Amidrest ein N-substituiertes Piperazin tragen.

Neben wirksamen und biologisch verfügbaren Thrombin-Hemmstoffen wurden in der vorgestellten Stoffklasse unter Derivaten, die am Piperazid-Stickstoff beispielsweise einen Heteroaryl- oder einen Acyl-Rest (-CO-X) tragen, bei dem X einen geradkettigen oder verzweigten Alkyl- ($C_3$-$C_{10}$), einen Aralkyl- oder einen Cycloalkyl-Rest ($C_3$-$C_{10}$) darstellt, bei Abnahme der Thrombinaktivität sehr wirksame Trypsin-Inhibitoren gefunden, die ebenfalls nach rektaler Applikation in erheblichem Umfang resorbiert werden. Die Ausschaltung der Trypsinaktivität durch Inhibitoren bei hyperproteolytischen Zuständen im Pankreas ist durchaus von therapeutischem Interesse.

GB-A-2 007 663 beschreibt als Antikoagulantien verwendbare $N^{\alpha}$alkyl- und -arylsulfonierte Amide von ω-Amidinophenyl-α-aminocarbonsäuren, u.a. von Amidinophenylalanin, wobei die Aminkomponente der Amidgruppe ein 5- bis 7-gliedriger heterocyclischer Ring sein kann, der zusätzlich zum mit der CO-Gruppe verknüpften Stickstoffatom ein weiteres oder weitere Heteroatom(e) enthalten und/oder substituiert sein kann.

EP-A-0 097 630 beschreibt thrombinsenkende $N^{\alpha}$-arylsulfonylierte Amide des p-Guanidinophenylalanins, wobei die Aminkomponente der Amidgruppe ein Heterocyclus sein kann, der zusätzlich zum mit der CO-Gruppe verknüpften Stickstoffatom ein Sauerstoffatom oder eine NH- oder eine $N-CH_3$-Gruppe enthalten kann.

WO-A-92/08709 beschreibt antithrombotisch wirksame $N^{\alpha}$-aryl- und -heteroarylsulfonylierte Ester und Amide des m-Amidino-, -Guanidino-, -Oxamidino-, -Aminomethyl- und -Aminophenylalanins, wobei als Aminokomponente einer Amidgruppe ein am zweiten Stickstoffatom gegebenenfalls durch niederes Alkyl, Aryl oder Alkoxycarbonyl substituiertes Piperazin in Frage kommt.

Die vorliegende Erfindung betrifft neue Proteinasen-hemmende Phenylalanin-piperazide der allgemeinen Formel I,

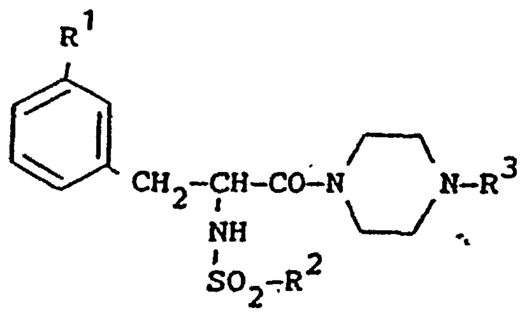

I

die als Racemate sowie als L- bzw. D-konfigurierte Verbindungen vorliegen können und in denen

$R^1$ eine basische Gruppe der Formel

(a) HN=C-NH$_2$ oder (b) -CH$_2$-NH$_2$

Amidino Aminomethyl

darstellt;

$R^2$ Phenyl, 4-Methyl-phenyl, 2,4,6-Trimethyl- bzw. -Triisopropyl-phenyl, 4-Methoxy-2,3,6-trimethyl-phenyl, 2,2-Dimethyl-6-methoxy- bzw. 2,2,5,7,8-Pentamethyl-chromanyl, Anthrachinonyl, 1- bzw. 2-Naphthyl, Chinolyl bzw. Isochinolyl oder einen Campherrest darstellt; und

$R^3$ einen Acylrest der Formel -COX darstellt, wobei X Wasserstoff, $C_{1-3}$-Alkyl oder $C_{3-10}$-Cycloalkyl darstellt; oder

einen Benzylrest darstellt, in dem der aromatische Rest gegebenenfalls mit einem Halogenatom oder einer $C_{1-3}$-Alkyl-, $C_{1-3}$-Alkoxy-, Hydroxy- oder Nitrogruppe substituiert ist; oder

einen Carbonsäureamidrest der Formel -CONR$^i$R$^{ii}$, einen Thiocarbonsäureamidrest der Formel -CSNR$^i$R$^{ii}$ oder einen Essigsäureamidrest der Formel -CH$_2$-CONR$^i$R$^{ii}$ darstellt, wobei $R^i = R^{ii} = H$; $R^i = R^i = C_{1-3}$-Alkyl; $R^i = H$, $R^{ii} = C_{1-3}$-Alkyl; $R^i = H$, $R^{ii}$ = Phenyl; oder $R^i$ und $R^{ii}$ zusammen mit dem Stickstoffatom einen 5- bis 7-gliedrigen Ring bilden, welcher ein weiteres Heteroatom enthalten kann; oder

einen Rest der Formel -SO$_2$-Y darstellt, in dem Y $C_{1-3}$-Alkyl, Trifluor-methyl, Trichlor-methyl, Phenyl, 4-Methyl-phenyl, 2,4,6-Trimethyl- bzw. -Triisopropyl-phenyl, 4-Methoxy-2,3,6-trimethyl-phenyl, 2,2-Dimethyl-6-methoxy- bzw. 2,2,5,7,8-Pentamethyl-chromanyl, Anthrachinonyl, Naphthyl, Chinolyl oder -NR$^{iii}$R$^{iv}$ bedeutet, wobei $R^{iii}$ und $R^{iv}$ jeweils H und/oder gleiches oder ungleiches $C_{1-3}$-Alkyl sind; oder

einen $C_{5-8}$-Cycloalkylring darstellt, der gegebenenfalls mit einer Hydroxyl- oder Oxogruppe substituiert sein kann; oder

einen Pyridyl-, Pyrimidinyl- oder N-Methylpiperidylrest darstellt; oder

einen funktionalisierten Alkylrest der Formel -(CH$_2$)$_n$-X' darstellt, wobei die Alkylkette unverzweigt oder verzweigt ist, n 1 bis 8 bedeutet und der funktionelle Rest X'

eine Hydroxygruppe darstellt, deren H-Atom gegebenenfalls durch $C_{1-3}$-Alkyl, Benzyl, Phenyl, 4-Methyl-phenyl, 2,4,6-Trimethyl- bzw. -Triisopropyl-phenyl, 4-Methoxy-2,3,6-trimethyl-phenyl, Anthrachinonyl, Naphthyl, Hydroxy-$C_{1-3}$-Alkyl oder durch eine Acylgruppe der obigen Formel -COX ersetzt ist, oder

ein Halogenatom bedeutet, oder

eine tertiäre Aminogruppe der Formel -NR$^v$R$^{vi}$ darstellt, wobei $R^v$ und $R^{vi}$ entweder gleiche $C_{1-3}$-Alkylgruppen sind oder zusammen mit dem Stickstoffatom einen 5- bis 7-gliedrigen Ring bilden, wobei gegebenenfalls ein oder zwei weitere Ringe annelliert ist/sind, oder

eine Acylaminomalonsäurediestergruppe der Formel

X-CO-HN-C(COO-$C_{1-3}$-Alkyl)$_2$,

worin X obige Bedeutung besitzt, eine Gruppe der Formel

$$X\text{-}CO\text{-}HN\text{-}CH\text{-}COOH,$$

worin X obige Bedeutung besitzt, oder eine Gruppe der Formel

$$H_2N\text{-}CH\text{-}COOH$$

darstellt;

und deren Salze mit Mineralsäuren oder organischen Säuren.

Von den in den vorstehend definierten Phenylalanin-piperaziden sind Verbindungen, bei denen $R^1$ eine basische Gruppe der Formel (a) = Amidino darstellt, $R^2$ eine 2-Naphthyl-, Anthrachinonyl-, 2,4,6-Triisopropylphenyl- oder eine 2,2,5,7,8-Pentamethylchromangruppe bedeutet und $R^3$ einen Acylrest, insbesondere Formyl und Acetyl, einen funktionalisierten Alkylrest, beispielsweise 2-Hydroxyethyl, einen $SO_2$-Y-Rest, einen Carbonsäureamidrest, 2-Pyridyl oder 2-Pyrimidyl darstellt, von besonderer Bedeutung.

Die Verbindungen der allgemeinen Formel I mit $R^1$ = Amidino (a) werden nach den nachfolgend beschriebenen, prinzipiell bekannten Methoden dargestellt.

(D,L)-3-Cyanphenylalanin-alkylester der allgemeinen Formel II werden in einem geeigneten Lösungsmittel mit einem Sulfochlorid der allgemeinen Formel III, in welcher $R^2$ die in der allgemeinen Formel I beschriebenen Bedeutungen besitzt, zu den racemischen Verbindungen der allgemeinen Formel IV umgesetzt, aus denen durch saure oder alkalische Hydrolyse die Racemate der sulfonylierten Cyanphenylalanine V erhalten werden.

Durch enzymatische Esterhydrolyse der Verbindungen IV mittels Chymotrypsin in einer Acetonitril/Wasser-Mischung sind die L-konfigurierten sulfonylierten Aminosäuren erhältlich. Die bei diesem Verfahren anfallenden sulfonylierten Aminocarbonsäure-alkylester mit D-Konfiguration IV werden durch saure Hydrolyse in einer Mischung aus 1 N HCl und Eisessig durch rückfliessendes Erhitzen in die D-konfigurierten sulfonylierten Aminocarbonsäuren V übergeführt.

Die Cyanverbindungen mit Piperazid-Struktur VI, die als Racemate bzw. in L- oder D-Form vorliegen können, sind nach üblichen Kupplungsverfahren aus den entsprechend konfigurierten Verbindungen V mit einem Piperazin-Derivat der allgemeinen Formel VII erhältlich. Ausserdem können die Piperazide VI auf prinzipiell bekannte Weise gewonnen werden, indem racemisches, L- oder D-3-Cyanphenylalanin zunächst durch Einführung einer Boc-Gruppe an der Aminofunktion geschützt wird. Die erhaltene Carbonsäure V, mit der Boc-Gruppe anstelle von $SO_2$-$R^2$, wird durch Umsetzung mit einem Piperazin-Derivat VII in eine entsprechende Boc-geschützte Verbindung VI übergeführt, aus der nach saurer Abspaltung der Boc-Gruppe und Umsetzung mit einem Sulfochlorid der allgemeinen Formel III die Cyanverbindungen mit Piperazid-Struktur VI erhalten werden.

Durch Addition von $H_2S$ an die Cyanfunktion können daraus die Thioamide VIII gewonnen werden, die durch Umsetzung mit einem Alkylhalogenid in die Thioimidsäureesterhalogenide IX übergeführt werden. Ausserdem können aus den Cyanverbindungen VI in bekannter Weise die Imidsäureesterhalogenide X erhalten werden.

Zur Darstellung der Zielverbindungen der allgemeinen Formel I mit $R^1$ = Amidino (a), die als Racemate bzw. in L-oder D-Form erhalten werden können und bei denen $R^2$ und $R^3$ die in der allgemeinen Formel I genannten Bedeutungen besitzen und X = Halogen, vorzugsweise Chlor, darstellt, werden die Thioimidsäureestersalze IX in alkoholischer Lösung mit Ammoniumacetat oder die Imidsäureestersalze X in alkoholischer Ammoniaklösung zu I umgesetzt. Die dabei erhaltenen Amidinsalze können in geeigneter Weise in die freien Basen übergeführt werden.

Die Verbindungen der allgemeinen Formel I mit $R^1$ = Aminomethyl (b) werden durch katalytische Hydrierung, beispielsweise Raney-Nickel/Wasserstoff in geeigneten Lösungsmitteln in Gegenwart von Ammoniak, aus den Cyanverbindungen VI erhalten.

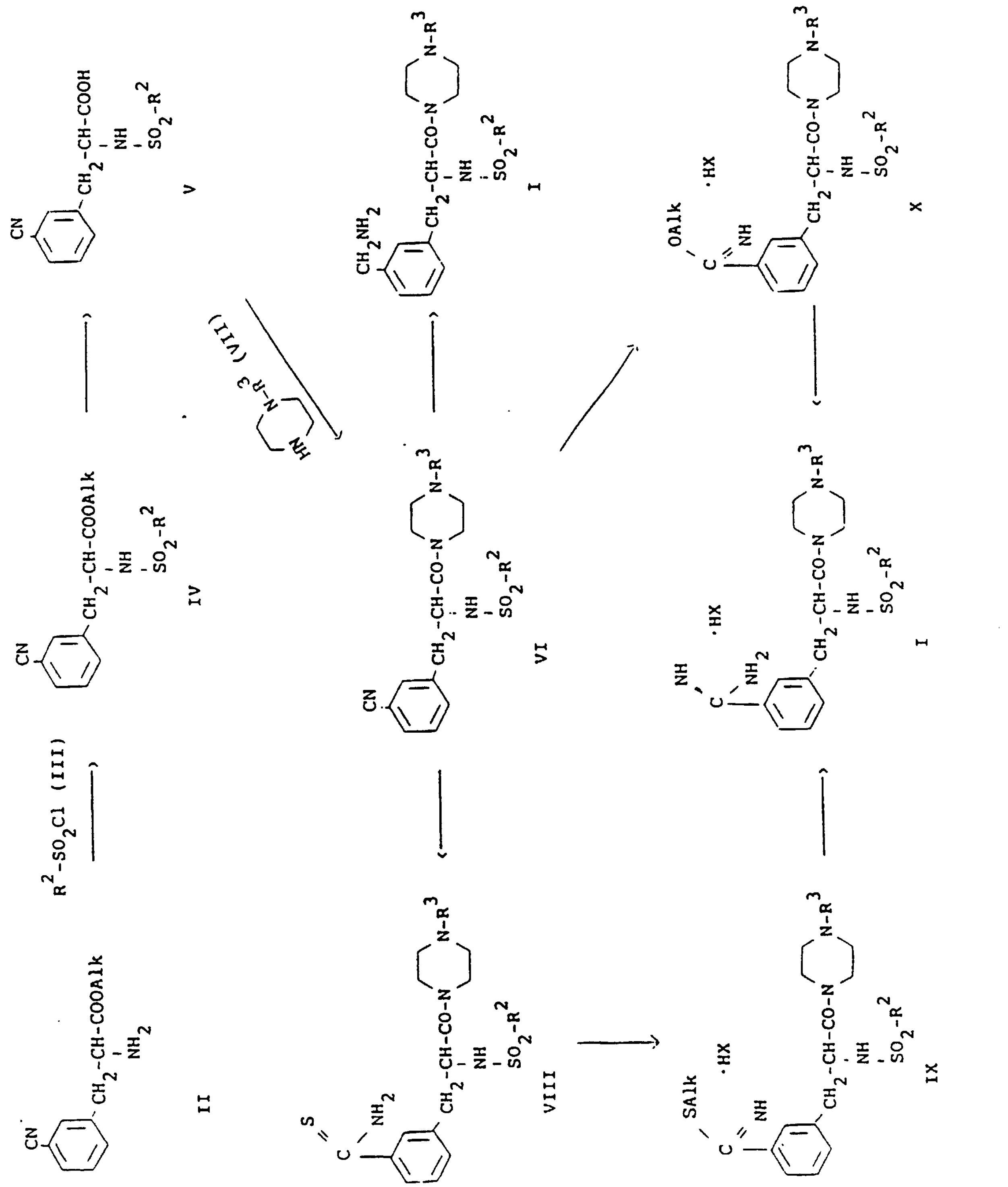


V I X
IV VI I
II VIII IX

Die biologische Aktivität der erfindungsgemässen Verbindungen wurde sowohl in vitro als auch in vivo bestimmt. Zur Charakterisierung der Inhibitoraktivität in vitro wurden die Dissoziationskonstanten $K_i$ für die Hemmung von Thrombin bzw. der verwandten Enzyme Trypsin, Plasmin, Faktor $X_a$, Faktor $XII_a$, Plasmakallikrein, glanduläres Kallikrein und tPA nach der Formel

$$K_i = \frac{[E] \cdot [I]}{[EI]}$$

bestimmt, in welcher [E] die Konzentration an freiem Enzym, [I] die Konzentration an freiem Inhibitor und [EI] die Konzentration an Enzym-Inhibitor-Komplex bezeichnen (Dixon, Biochem. J. 55, 170-173 [1953]). Je kleiner der $K_i$-Wert für ein geprüftes Enzym ist, desto grösser ist die Affinität des Inhibitors für das Enzym und desto kleiner ist die zur Hemmung des Enzyms, z.B. Thrombin, benötigte Menge Inhibitor.

In vitro wurden verschiedene Gerinnungstests benutzt, um die Wirksamkeit der Hemmstoffe gegenüber der durch Thrombin ausgelösten Gerinnung seines natürlichen Substrates Fibrinogen zu bestimmen. Dazu wurde in Human-Plasma die Thrombinzeit (TT), die aktivierte partielle Thromboplastinzeit (aPTT) und die Prothrombinzeit (PT, Quickwert) bestimmt.

Die Toxizität der erfindungsgemässen Verbindungen wurde durch Bestimmung der $LD_{50}$ (= Dosis, die bei 50% der Versuchstiere während einer Beobachtungsdauer von einer Woche zum Tode führt) an der Maus nach intravenöser bzw. peroraler Verabreichung ermittelt.

Zur pharmakokinetischen Charakterisierung wurde die Plasmakonzentration ausgewählter Derivate nach intravenöser (i.v.), peroraler (p.o.), intraduodenaler (i.d.) und rektaler Applikation an Ratten nach folgendem dreistufigem Verfahren bestimmt:

1. Eine Lösung der zu prüfenden Substanz in physiologischer Kochsalzlösung wurde der Flüssigkeits-Hochdruckchromatographie (HPLC = high pressure liquid chromatography) unterworfen, um den für die Substanz charakteristischen Peak bei der unter den gewählten Versuchsbedingungen substanzspezifischen Retentionszeit zu ermitteln.
2. Die zu prüfende Substanz wurde in vitro in Rattenplasma gelöst. Diese Lösung wurde ebenfalls der HPLC unterworfen, um festzustellen, ob der für die Substanz charakteristische Peak bei der substanzspezifischen Retentionszeit erneut erscheinen würde.
3. Die zu prüfende Substanz wurde in physiologischer Kochsalzlösung gelöst und in einer Dosis von 1, 50 bzw. 100 mg pro kg Körpergewicht i.v., p.o., i.d. bzw. rektal an Ratten verabreicht. In Zeitintervallen von 15 Minuten wurden Blutproben entnommen, aus denen durch Zentrifugation Plasmaproben hergestellt wurden, welche ihrerseits der HPLC unterworfen wurden, um festzustellen, ob der für die Substanz charakteristische Peak bei der substanzspezifischen Retentionszeit wiederum in Erscheinung treten würde.

Zum Nachweis der pharmakologischen Wirksamkeit wurde die zu prüfende Substanz in physiologischer Kochsalzlösung gelöst und in einer Dosis von 5 bzw. 20 mg pro kg Körpergewicht rektal an Ratten verabreicht. In Zeitintervallen wurden Blutproben entnommen, aus denen durch Zentrifugation Plasmaproben hergestellt und im Gerinnungstest (Thrombinzeit TT und aktivierte partielle Thromboplastinzeit aPTT) geprüft wurden.

Die antithrombotische Aktivität der Verbindungen wurde am Modell der Stase-induzierten Thrombose bei der Ratte nach Wessler et al. (J. Appl. Physiol. 14, 943-946, 1959) bestimmt. Der Thrombus wurde 30 min nach Applikation des Hemmstoffs durch Serum induziert und nach weiteren 10 min makroskopisch bewertet.

Die erfindungsgemässen Verbindungen können als Diagnostika oder in geeigneter Applikationsform in Gestalt ihrer Salze oder der freien Basen als Arzneimittel zur Anwendung kommen.

Die Erfindung soll an vier Ausführungsbeispielen näher erläutert werden.

Beispiel 1

Nα-(2-Naphthylsulfonyl)-3-amidino-(L)-phenylalanin-4-acetyl-piperazid

Nα-(2-Naphthylsulfonyl)-3-cyan-(D,L)-phenylalaninmethylester (Formel IV; Alk = $-CH_3$, $R^2$ = β-Naphthyl)

24.1 g (0.1 mol) 3-Cyan-(D,L)-phenylalaninmethylester-hydrochlorid wurden in 200 ml Dioxan suspendiert, unter Rühren 20.6 g (0.204 mol) N-Methylmorpholin zugefügt und eine Lösung von 23.6 g (0.104 mol) 2-Naphthylsulfonylchlorid in 200 ml Ethylacetat zugetropft. Es wurde 16 Std. bei Raumtemperatur gerührt, ausgefallenes N-Methylmorpholin-hydrochlorid abfiltriert und das Lösungsmittel abdestilliert. Der Rückstand wurde in 50 ml Methanol gelöst, 50 ml Diethylether zugegeben und zur Kristallisation stehengelassen. Der gebildete Niederschlag wurde abgesaugt, mit Diethylether gewaschen und im Vakuum-Exsikkator ($KOH/H_2SO_4$) getrocknet. Ausbeute: 36 g (91.3%). Smp.: 122-123°C.
DC: $R_f$ = 0.65 (Chloroform 40/Methanol 4/Eisessig 1//v/v/v/)

Nα-(2-Naphthylsulfonyl)-3-cyan-(L)-phenylalanin (Formel V; $R^2$ = β-Naphthyl)

17.4 g (0.044 mol) Nα-(2-Naphthylsulfonyl)-3-cyan-(D,L)-phenylalaninmethylester wurden in 260 ml Acetonitril gelöst, 130 ml Wasser, 100 mg Chymotrypsin sowie 0.785 g Kaliumchlorid zugefügt und der pH-Wert der Lösung

mit 2 N NaOH auf 6.8-7 gestellt. Der Ansatz wurde 24 Std. bei Raumtemperatur gerührt, wobei nach 5 und 10 Std. noch jeweils 50 mg Chymotrypsin zugegeben und der oben angegebene pH-Wert der Lösung durch kontrollierte Zugabe von insgesamt 11 ml 2 N NaOH gehalten wurde. Anschliessend wurde filtriert, das Acetonitril im Vakuum abdestilliert und die wässrige Lösung mehrfach mit Ethylacetat extrahiert. Nach Ansäuern der wässrigen Phase mit 1 N HCl wurde diese wiederholt mit Ethylacetat ausgeschüttelt, die vereinigten organischen Phasen mit gesättigter Natriumchloridlösung gewaschen, über Magnesiumsulfat getrocknet und das Lösungsmittel abdestilliert, wobei gegen Ende der Destillation das Produkt auszukristallisieren begann. Die Destillation wurde unterbrochen, 30 ml Diethylether und 70 ml Hexan zugefügt, der Niederschlag abgesaugt, mit wenig Diethylether gewaschen und getrocknet. Ausbeute: 7.2 g (85.8%). Smp.: 192-193°C. $[\alpha]_D^{20}$ = + 11.9° (C = 3, in Methanol)
DC: $R_{-f}$ = 0.25 (Chloroform 40/Methanol 4/Eisessig 1//v/v/v)

Na-(2-Naphthylsulfonyl)-3-cyan-(L)-phenylalanin-4-acetylpiperazid (Formel VI; $R^2$ = β-Naphthyl, $R^3$ = $-COCH_3$)

1.59 g (13.2 mmol) 1-Acetylpiperazin wurden in 10 ml THF und 20 ml DMF gelöst, die Lösung mit 1.28 g (7.9 mmol) HOBt und 2.5 g (6.6 mmol) Nα-(2-Naphthylsulfonyl)-3-cyan-(L)-phenylalanin versetzt und auf 0°C gekühlt. Nach Zugabe von 1.5 g (7.3 mmol) DCC wurde noch 2 Std. bei 0°C und anschliessend 22 Std. bei Raumtemperatur gerührt. Der ausgefallene Dicyclohexylharnstoff wurde abfiltriert und das Lösungsmittel im Vakuum abdestilliert. Der Rückstand wurde in Chloroform gelöst und säulenchromatographisch über Kieselgel 60 mit Chloroform/Methanol 93:7 als Eluierungsmittel gereinigt. Man erhielt 2.9 g (90%) eines amorphen Produktes.
$[\alpha]_D^{20}$ = + 46.3° (C = 1, in Methanol)
DC: $R_f$ = 0.36 (Chloroform 40/Methanol 4/Eisessig 1//v/v/v)

Nα-(2-Naphthylsulfonyl)-3-thiocarboxamido-(L)-phenylalanin-4-acetylpiperazid (Formel VIII; $R^2$ = β-Naphthyl, $R^3$ = $-COCH_3$)

2.75 g (5.6 mmol) Nα-(2-Naphthylsulfonyl)-3-cyan-(L)-phenylalanin-4-acetylpiperazid wurden in 25 ml Pyridin gelöst, die Lösung mit 20 Tropfen TEA versetzt und durch 10-min. Einleiten mit $H_2S$ gesättigt. Der Ansatz wurde 2 Tage bei Raumtemperatur stehengelassen, anschliessend das Lösungsmittel abdestilliert, der Rückstand in Ethylacetat gelöst und mit 1 N HCl ausgeschüttelt. Die organische Phase wurde mit gesättigter Natriumchloridlösung gewaschen, über Magnesiumsulfat getrocknet und das Lösungsmittel abdestilliert. Es wurden 2.6 g (88%) gelbes, amorphes Produkt erhalten, das in dieser Form weiterverarbeitet wurde.

Nα-(2-Naphthylsulfonyl)-3-S-methyliminothiocarbonyl-(L)-phenylalanin-4-acetylpiperazid-hydroiodid (Formel IX; Alk = $-CH_3$, X = I, $R^2$ = β-Naphthyl, $R^3$ = $-COCH_3$)

2.6 g (4.96 mmol) des vorstehend beschriebenen Thioamids wurden in 60 ml Aceton gelöst, die Lösung mit 6 g (42.3 mmol) Methyliodid versetzt und der Ansatz 20 Std. bei Raumtemperatur unter Lichtschutz stehengelassen. Danach wurde das Lösungsmittel abdestilliert, der ölige Rückstand mit Isopropanol/Diethylether durchgearbeitet, das dabei erhaltene Pulver abgesaugt, mit Diethylether gewaschen und getrocknet. Ausbeute: 3.1 g (94%).

Nα-(2-Naphthylsulfonyl)-3-amidino-(L)-phenylalanin-4-acetylpiperazid-hydrochlorid (Formel I; X = Cl, $R^2$ = β-Naphthyl, $R^3$ = $-COCH_3$)

3.0 g (4.5 mmol) Thioimidsäuremethylester-hydroiodid wurden in 100 ml Methanol gelöst, die Lösung mit 0.8 g (10.4 mmol) Ammoniumacetat versetzt und der Ansatz 3 Std. bei 60°C im Wasserbad erwärmt. Anschliessend wurde das Lösungsmittel abdestilliert, der Rückstand in heissem Isopropanol gelöst und das Amidinhydroiodid mit Ethylacetat ausgefällt, abgesaugt, mit Ethylacetat und Diethylether gewaschen und getrocknet. Zur Überführung in das Hydrochlorid wurde das erhaltene Produkt in Methanol gelöst und die Lösung über einen stark basischen Ionenaustauscher (Amberlite IRA-410, $Cl^-$ beladen) gegeben. Das Hydrochlorid wurde aus der eingeengten methanolischen Lösung mit Ethylacetat/Diethylether 1:1 ausgefällt. Ausbeute: 1.8 g (73.5%). $[\alpha]_D^{20}$ = + 61.8° (C = 1, in Methanol)
DC: $R_f$ = 0.2 (organische Phase von Ethylacetat 4/Eisessig 1/Wasser 2//v/v/v)
Die spezifische Drehung der entsprechenden (D)-konfigurierten Verbindung: $[\alpha]_D^{20}$ = - 62.2° (C = 1, in Methanol)

Beispiel 2

Nα-(2-Naphthylsulfonyl)-3-amidino-(L)-phenylalanin-4-(2-hydroxyethyl)-piperazid

Nα-(2-Naphthylsulfonyl)-3-cyan-(L)-phenylalanin-4-(2-hydroxyethyl)-piperazid-hydrochlorid (Formel VI; $R^2$ = β-Naphthyl, $R^3$ = $-CH_2CH_2OH$)

2.0 g Nα-(2-Naphthylsulfonyl)-3-cyan-(L)-phenylalanin wurden in 10 ml Thionylchlorid eingetragen und der Ansatz 30 Min. unter Rückfluss erhitzt. Die erhaltene Lösung wurde nach dem Erkalten mit Hexan bis zur starken Trübung versetzt, nach 1 Std. das auskristallisierte Säurechlorid abgesaugt, mit Hexan gewaschen und im Vakuum getrocknet. 1.7 g (4.26 mmol) dieses Produktes wurden in 25 ml THF gelöst und im Verlauf von 15 Min. unter Rühren in eine Lösung von 1.16 g (8.9 mmol) 1-(2-Hydroxyethyl)-piperazin in 15 ml THF getropft. Es wurde noch 1 Std. gerührt, anschliessend ausgefallenes 1-(2-Hydroxyethyl)-piperazin-hydrochlorid abfiltriert und das Lösungsmittel abdestilliert. Der verbleibende Rückstand wurde in 15 ml Methanol gelöst, Wasser bis zur starken Trübung hinzugefügt und über Nacht stehengelassen, wobei sich das Piperazid als Öl abgeschieden hatte. Nach Abgiessen des überstehenden Lösungsmittels wurde das Öl in 100 ml Ethylacetat aufgenommen, die Ethylacetatlösung mit gesättigter Natriumchloridlösung gewaschen, über $MgSO_4$ getrocknet und das Lösungsmittel zur Hälfte abdestilliert. Die verbleibende Lösung wurde mit 2 N Ethylacetat/HCl angesäuert, 50 ml Diethylether zugefügt, der gebildete Niederschlag nach 1 Std. Stehen abgesaugt, mit Diethylether gewaschen und im Vakuum getrocknet. Ausbeute: 1.65 g (73%). $[\alpha]_D^{20}$ = - 5.4° (C = 1, in Methanol)
DC: $R_f$ = 0.43 (organische Phase von Ethylacetat 4/Eisessig 1/Wasser 2//v/v/v)

Nα-(2-Naphthylsulfonyl)-3-methoxyiminocarbonyl-(L)-phenylalanin-4-(2-hydroxyethyl)-piperazid-dihydrochlorid (Formel X; Alk = $-CH_3$, X = Cl, $R^2$ = β-Naphthyl, $R^3$ = $-CH_2CH_2OH$)

1.4 g (2.65 mmol) der vorher beschriebenen Cyanverbindung wurden in einer Mischung aus 7.5 ml abs. Methanol und 10 ml abs. Dioxan gelöst, in die Lösung unter Eiskühlung 5.2 g (0.143 mol) getrocknetes HCl-Gas eingeleitet und der Ansatz 3 Tage im Kühlschrank aufbewahrt. Anschliessend wurde in 150 ml Diethylether gegossen, der gebildete Niederschlag nach Abgiessen des überstehenden Lösungsmittels mit 40 ml abs. Ethanol durchgearbeitet, das Kristallpulver abgesaugt, mit Ethanol und Diethylether gewaschen und getrocknet. Ausbeute: 1.44 g (91%).

DC: $R_f$ = 0.15 (organische Phase von Ethylacetat 4/Eisessig 1/Wasser 2//v/v/v)
DC: $R_f$ = 0.95 (Chloroform 70/Methanol 42/Eisessig 0.5/Wasser 10//v/v/v)

Na-(2-Naphthylsulfonyl)-3-amidino-(L)-phenylalanin-4-(2-hydroxyethyl)-piperazid-dihydrochlorid (Formel I; X = Cl, $R^2$ = β-Naphthyl, $R^3$ = $-CH_2CH_2OH$)

1.3 g (2.18 mmol) des davor beschriebenen Imidsäuremethylester-dihydrochlorids wurden in 30 ml Methanol suspendiert und unter Rühren bis zu einem pH-Wert von 8.7 ethanolische Ammoniaklösung zugefügt, wobei eine klare Lösung erhalten wurde. Der Ansatz wurde 3 Std. bei 60°C im Wasserbad erwärmt, anschliessend das Lösungsmittel abdestilliert, der Rückstand in 15 ml abs. Ethanol gelöst und nach Zugabe von 20 Tropfen 2 N Ethylacetat/HCl das Amidin-dihydrochlorid mit Ethylacetat ausgefällt, abgesaugt, mit Ethylacetat und Diethylether gewaschen und getrocknet. Ausbeute: 1.02 g (80.3%). $[\alpha]_D^{20}$ = + 14.2° (C = 1, in Methanol)

DC: $R_f$ = 0.18 (organische Phase von Ethylacetat 4/Eisessig 1/Wasser 2//v/v/v)
DC: $R_f$ = 0.6 (Chloroform 70/Methanol 42/Eisessig 0.5/Wasser 10//v/v/v)

Die spezifische Drehung der entsprechenden (D)-konfigurierten Verbindung: $[\alpha]_D^{20}$ = - 15.0° (C = 1, in Methanol)

Zur Gewinnung der freien Base wurden 0.5826 g (1 mmol) Amidindihydrochlorid in 20 ml Methanol gelöst, die Lösung mit der äquimolaren Menge 0.1 N NaOH (20.00 ml) versetzt und das Lösungsmittel abdestilliert. Zur Entfernung noch vorhandener Wasserspuren wurde mehrfach mit Toluol/Isopropanol kodestilliert. Die auf diese Weise erhaltene Base enthält noch NaCl. Zur Beseitigung des anorganischen Bestandteiles wurde eine Mischung aus 15 ml abs. Ethanol und je 10 ml Chloroform und Diethylether zugefügt und gerührt, wobei sich die Base löste. Ungelöstes NaCl wurde abfiltriert und das Lösungsmittel abdestilliert. Der verbleibende Rückstand wurde beim Anreiben mit Diethylether fest.
Ausbeute: 0.48 g (94%)

Beispiel 3

N$\alpha$-(2-Naphthylsulfonyl)-3-amidino-(L)-phenylalanin-4-methylsulfonylpiperazid

N$\alpha$-(2-Naphthylsulfonyl)-3-cyan-(L)-phenylalanin-4-methylsulfonylpiperazid (Formel VI, $R^2$ = $\beta$-Naphthyl, $R^3$ = $-SO_2CH_3$) 1.56 g (7.8 mmol) 1-Methylsulfonylpiperazin · HCl wurden in 15 ml DMF suspendiert, die Suspension unter Rühren mit 0.86 ml (7.8 mmol) NMM, 1.16 g (7.8 mmol) HOBt, 2.7 g N$\alpha$-(2-Naphthylsulfonyl)-3-cyan-(L)-phenylalanin (7.1 mmol) sowie 70 ml THF versetzt und auf 0°C gekühlt. Nach Zugabe von 1.61 g DCC (7.8 mmol) wurde noch 20 Std. bei Raumtemperatur gerührt. Danach wurde der ausgefallene Dicyclohexylharnstoff abgesaugt und das Lösungsmittel im Vakuum abdestilliert. Der Rückstand wurde in Chloroform gelöst und säulenchromatographisch über Kieselgel 60 mit Chloroform als Eluierungsmittel gereinigt. Es wurden 3.34 g (89%) eines amorphen Produktes erhalten.
$[\alpha]_D^{20}$ = + 47.3° (C = 1, in Methanol)
DC: $R_f$ = 0.36 (Chloroform 40/Methanol 4/ Eisessig 1//v/v/v)

N$\alpha$-(2-Naphthylsulfonyl)-3-thiocarboxamido-(L)-phenylalanin-4-methylsulfonylpiperazid (Formel VIII; $R^2$ = $\beta$ -Naphthyl, $R^3$ = $SO_2CH_3$)

2.9 g (5.2 mmol) der vorstehend beschriebenen Cyanverbindung wurden in 35 ml Pyridin gelöst, 15 Tropfen TEA zugefügt und die Lösung durch 10-min. Einleiten mit $H_2S$ gesättigt. Der Ansatz wurde 2 Tage bei Raumtemperatur stehengelassen, anschliessend das Lösungsmittel abdestilliert und der Rückstand in Ethylacetat gelöst, wobei das Thioamid allmählich auskristallisierte. Der Niederschlag wurde abgesaugt, mit Ethylacetat gewaschen und getrocknet. Ausbeute: 2.98 g (96%).

N$\alpha$-(2-Naphthylsulfonyl)-3-S-methyliminothiocarbonyl-(L)-phenylalanin-4-methylsulfonylpiperazid (Formel IX; Alk = $CH_3$, X = I, $R^2$ = $\beta$-Naphthyl, $R^3$ = $-SO_2CH_3$)

2.95 g (5.26 mmol) des beschriebenen Thioamids wurden unter Erwärmen in 4 ml DMF gelöst, die Lösung mit 50 ml Aceton und 7.1 g (50 mmol) Methyliodid versetzt und der Ansatz 20 Std. bei Raumtemperatur unter Lichtschutz aufbewahrt. Anschliessend wurde in 400 ml Diethylether gegossen, der gebildete Niederschlag abgesaugt, mit Diethylether gewaschen und getrocknet.
Ausbeute: 3.25 g (88%).

N$\alpha$-(2-Naphthylsulfonyl)-3-amidino-(L)-phenylalanin-4-methylsulfonylpiperazid-hydrochlorid (Formel I; X = Cl, $R^2$ = $\beta$-Naphthyl, $R^3$ = $-SO_2CH_3$)

3.23 g (4.6 mmol) Thioimidsäuremethylester-hydroiodid wurden in 110 ml Methanol gelöst, die Lösung mit 0.58 g (7.5 mmol) Ammoniumacetat versetzt und der Ansatz 3 Std. bei 60°C im Wasserbad erwärmt. Anschliessend wurde das Lösungsmittel abdestilliert, der Rückstand in Methanol gelöst und das Amidinhydroiodid mit Ethylacetat/ Diethylether 9:1 ausgefällt, abgesaugt, mit Diethylether gewaschen und getrocknet. Zur Überführung in das Hydrochlorid wurde das erhaltene Produkt in Methanol gelöst und die Lösung über einen stark basischen Ionenaustauscher (Amberlite IRA-410, Cl-beladen) gegeben. Das Hydrochlorid wurde aus der eingeengten methanolischen Lösung mit Diethylether ausgefällt.
Ausbeute: 2.1 g (79%). $[\alpha]_D^{20}$ = + 70.0° (C = 1, in Methanol).
DC: $R_f$ = 0.32 (organische Phase von Ethylacetat 4/Eisessig 1/Wasser 2//v/v/v)
Die spezifische Drehung der entsprechenden (D)-konfigurierten Verbindung: $[\alpha]_D^{20}$ = - 70.5° (C = 1, in Methanol)

Beispiel 4

Pmc-3-amidino-(L)-phenylalanin-4-methylsulfonylpiperazid

Boc-3-cyan-(L)-phenylalanin

6 g (26.5 mmol) 3-Cyan-(L)-phenylalanin-hydrochlorid und 9.1 ml (53.2 mmol) N-Ethyldiisopropylamin wurden in 17 ml Wasser suspendiert, die Suspension mit einer Lösung von 7.2 g (29.2 mmol) 2-(Boc-oxyimino)-2-phenylacetonitril in 20 ml Dioxan versetzt und 16 Std. bei Raumtemperatur gerührt. Nach anschliessender Zugabe von 50 ml Wasser wurde die Lösung mit 50 ml Ethylacetat ausgeschüttelt, die organische Phase abgetrennt und die wässrige Phase mit verdünnter Salzsäure auf pH 3 gebracht. Es wurde 3 mal mit je 100 ml Ethylacetat extrahiert, die vereinigten organischen Lösungen mit ges. Natriumchloridlösung gewaschen, über Magnesiumsulfat getrocknet und das Lösungsmittel abdestilliert.
Ausbeute: 6.2 g (81%)

Boc-3-cyan-(L)-phenylalanin-4-methylsulfonylpiperazid

4.92 g (24.5 mmol) 1-Methylsulfonylpiperazin-hydrochlorid und 2.7 ml (24.5 mmol) NMM wurden in 50 ml DMF gelöst, 4 g (29.6 mmol) HOBt sowie eine Lösung von 5.93 g (20.4 mmol) Boc-3-cyan-(L)-phenylalanin in 200 ml THF zugefügt und der Ansatz auf 0°C abgekühlt. Nach Zugabe von 5.1 g (24.7 mmol) DCC wurde 48 Std. bei Raumtemperatur gerührt. Danach wurde der ausgefallene Dicyclohexylharnstoff abgesaugt, der THF-Anteil der Lösung im Vakuum abdestilliert, filtriert und das Filtrat in eine Mischung aus 100 ml 5proz. Natriumbicarbonatlösung und 200 ml Eiswasser gegossen. Der ausgefallene Niederschlag wurde abgesaugt, mit Wasser gewaschen, in Methanol gelöst und das Lösungsmittel abdestilliert. Zur Entfernung des noch vorhandenen Wassers wurde mehrfach mit Toluol/Isopropanol kodestilliert. Das nur geringfügig verunreinigte Produkt wurde in der Form weiterverarbeitet. Ausbeute: 7.9 g (89%)

3-Cyan-(L)-phenylalanin-4-methylsulfonylpiperazid-hydrochlorid

7.9 g des vorstehend beschriebenen Rohproduktes wurden in 70 ml Ethylacetat und 30 ml Diethylether gelöst, die Lösung mit 50 ml 2.5 N HCl in Ethylacetat versetzt und die Lösung 48 Std. bei Raumtemperatur gerührt, wobei das gewünschte Hydrochlorid auskristallisierte. Anschliessend wurden noch 200 ml Diethylether zugefügt, 1 Std. gerührt, der Niederschlag abgesaugt, mit Diethylether gewaschen und getrocknet. Ausbeute: 4.95 g (73%).

Pmc-3-cyan-(L)-phenylalanin-4-methylsulfonylpiperazid (Formel VI; $R^2$ = -Pmc, $R^3$ = $-SO_2CH_3$)

4.76 g (12.8 mmol) 3-Cyan-(L)-phenylalanin-4-methylsulfonyl-piperazid-hydrochlorid und 1.29 g (12.8 mmol) NMM wurden in 25 ml DMF gelöst, 4.64 g (15.3 mmol) Pmc-Chlorid und 1.55 g (15.3 mmol) NMM zugefügt und 48 Std. bei Raumtemperatur gerührt. Anschliessend wurde ausgefallenes NMM-hydrochlorid abfiltriert und das Lösungsmittel im Vakuum abdestilliert. Der Rückstand wurde in Ethylacetat aufgenommen und die organische Phase mit 0.1 N HCl sowie ges. Natriumchloridlösung gewaschen. Nach Trocknen über Magnesiumsulfat wurde das Lösungsmittel abdestilliert. Das erhaltene Produkt wurde säulenchromatographisch über Kieselgel 60 mit Chloroform als Eluierungsmittel gereinigt.
Ausbeute: 6.45 g (84%).
$[\alpha]_D^{20}$ = + 10.4° (C = 1, in Methanol)
DC: $R_f$ = 0.66 (Chloroform 40/ Methanol 4/Eisessig 1//v/v/v)

Pmc-3-thiocarboxamido-(L)-phenylalanin-4-methylsulfonylpiperazid (Formel VII; $R^2$ = -Pmc, $R^3$ = $-SO_2CH_3$)

6.34 g (10.5 mmol) der vorstehende beschriebenen Cyanverbindung wurden in 40 ml Pyridin gelöst, 20 Tr. TEA zugefügt und die Lösung durch 10-min. Einleiten mit $H_2S$ gesättigt. Der Ansatz wurde 2 Tage bei Raumtemperatur stehengelassen, anschliessend das Lösungsmittel abdestilliert, der feste Rückstand in 100 ml Ethylacetat suspendiert, kurz aufgekocht, abgesaugt, mit Ethylacetat gewaschen und getrocknet.
Ausbeute: 5.86 g (88%).

Pmc-3-S-methyliminothiocarbonyl-(L)-phenylalanin-4-methylsulfonylpiperazid (Formel IX; Alk = $-CH_3$, X = I, $R^2$ = -Pmc, $R^3$ = $-SO_2CH_3$)

5.86 g (7.52 mmol) des vorher beschriebenen Thioamids wurden unter Erwärmen in 11 ml DMF gelöst, 250 ml Aceton und 13 g (92 mmol) Methyliodid zugefügt und der Ansatz über Nacht bei Raumtemperatur unter Lichtschutz aufbewahrt. Anschliessend wurde in 1 1 Diethylether gegossen, 1 Std. gerührt, der gebildete Niederschlag abgesaugt, mit Diethylether gewaschen und getrocknet.
Ausbeute: 6.23 g (87%).

Pmc-3-amidino-(L)-phenylalanin-4-methylsulfonylpiperazidhydrochlorid (Formel I; X = Cl, $R^2$ = -Pmc, $R^3$ = $-SO_2CH_3$)

6.23 g (8 mmol) Thioimidsäureesterhydroiodid wurden in 350 ml abs. Methanol gelöst, zunächst 1 g (13 mmol) Ammoniumacetat zugefügt und der Ansatz bei 60°C im Wasserbad unter Rühren erwärmt, wobei sich zunächst ein Niederschlag abschied, der sich erst nach 4 Std. wieder vollständig gelöst hatte. Der Reaktionsverlauf wurde dünnschichtchromatographisch verfolgt und nach 2, 4 bzw. 6 Std. noch insgesamt 1 g (13 mmol) Ammoniumacetat in Portionen von 0.2, 0.5 und 0.3 g zugefügt. Nach 8 Std. war dünnschichtchromatographisch keine Ausgangsverbindung mehr nachzuweisen. Das Lösungsmittel wurde jetzt abdestilliert, der Rückstand in Ethanol gelöst und das Amidinhydroiodid mit Ethylacetat/Diethylether 1:1 ausgefällt. Zur Überführung in das Hydrochlorid wurde das erhaltene Produkt in Methanol gelöst und die Lösung über einen stark basischen Ionenaustauscher (Amberlite IRA-410, $Cl^-$ beladen) gegeben. Das Hydrochlorid wurde aus der eingeengten methanolischen Lösung mit Diethylether ausgefällt.
Ausbeute: 3.34 g (64%). $[\alpha]_D^{20}$ = + 47.7° (C = 1, in Methanol).
DC: $R_f$ = 0.5 (organische Phase von Ethylacetat 4/Eisessig 1/Wasser 2//v/v/v)

Die spezifische Drehung der entsprechenden (D)-konfigurierten Verbindung: $[\alpha]_D^{20}$ = - 48.3° (C = 1, in Methanol). Alle Amidinhydrochloride wurde säulenchromatographisch über Sephadex LH-20 mit Methanol als Eluierungsmittel gereinigt.

Abkürzungen

| | |
|---|---|
| Ac | Acetyl |
| AcONHiPr | $-CH_2CONHCH(CH_3)_2$ |
| BOC | t-Butyloxycarbonyl |
| Bzl | Benzyl |
| cBu | Cyclobutyl |
| cHex | Cyclohexyl |
| cPr | Cyclopropyl |
| DCC | Dicyclohexylcarbodiimid |
| DMF | Dimethylformamid |
| Et | Ethyl |
| EtOH | Hydroxyethyl |
| EtOEtOH | Hydroxyethyl-ethoxy |
| For | Formyl |
| HOBt | Hydroxybenzotriazol |
| Me | Methyl |
| NMM | N-Methylmorpholin |
| Ph | Phenyl |
| TEA | Triethylamin |
| THF | Tetrahydrofuran |

<u>LEGENDE ZU DEN TABELLEN 1 - 2</u>

| $R_1$ | $R_2$ |
|---|---|
| AMD = Amidino | Mtr = 2,3,6-Methyl-4-methoxy-phenyl |
| AMe = Aminomethyl | TIPP = 2,4,6-Triisopropyl-phenyl |
| | TMeP = 2,4,6-Trimethyl-phenyl |
| | Tol = 4-Methyl-phenyl |
| | 2-Naph = ß-Naphthyl |
| | 1-Naph = α-Naphthyl |

Pmc = 2,2,5,7,8-Pentamethylchroman 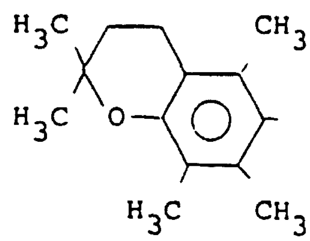

Cm = Campher-10 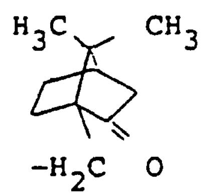

AC = Anthrachinon 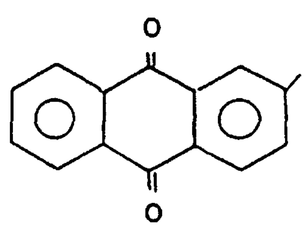

$R_3$

2-Pyl = 2-Pyridyl 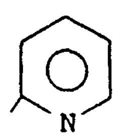 2-Pym = 2-Pyrimidyl 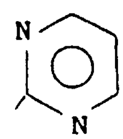

Im folgenden sind die biologischen Eigenschaften von repräsentativen erfindungsgemässen Verbindungen aufgeführt:

In Tabelle 1 ist die Hemmung des Gerinnungsenzyms Thrombin im Vergleich zu Trypsin anhand der Dissoziationskonstante $K_i$ (ausgedrückt in µmol/l) durch die genannten Verbindungen angegeben. Alle untersuchten Verbindungen hemmen die durch beide Enzyme bewirkte Substratspaltung kompetitiv. Unter den in Tabelle 1 aufgeführten Derivaten des 3-Amidinophenylalanins finden sich eine Reihe von Verbindungen mit hoher Antithrombinaktivität, d.h. mit $K_i$-Werten zwischen 0.1 und 0.001 µmol/l. Die Thrombinhemmung ist bei der Mehrzahl der Verbindungen stärker als die Hemmung von Trypsin. Die $K_i$-Werte für die Hemmung von Trypsin liegen bis zu einer Grössenordnung höher als die für die Thrombinhemmung. Eine Reihe von Verbindungen hemmt allerdings Thrombin und Trypsin mit vergleichbarer Affinität, einige Derivate, insbesondere solche mit bestimmten Acyl- oder Heteroaryl-Resten am Piperazin-Stickstoff sind wirksame Trypsinhemmstoffe.

Darüberhinaus wird in Tabelle 1 auch die Hemmwirkung gegenüber Plasmin, Faktor $X_a$, Faktor $XII_a$, Plasmakallikrein, glandulärem Kallikrein und tPA dargestellt. Gewöhnlich werden Plasmin, Faktor $X_a$ und Plasmakallikrein sehr viel schwächer gehemmt, die $K_i$-Werte sind 1-2 Grössenordnungen höher. Praktisch unwirksam sind die Derivate gegenüber Faktor $XII_a$, tPA und glandulärem Kallikrein. Für eine Reihe von Verbindungen kann man daher von selektiven Thrombinhemmstoffen sprechen, andere Derivate bevorzugen Trypsin.

Die Toxizität bei einer Reihe von erfindungsgemässen Verbindungen liegt in der gleichen Grössenordnung wie bei früher geprüften Derivaten von Benzamidin-enthaltenden Aminosäuren ($LD_{50}$ 10 - 50 mg/kg nach i.v.-Applikation).

Bei einigen Derivaten wurden die optischen Antipoden dargestellt und auf ihre Hemmwirkung geprüft. Entsprechend den Befunden von Turk, D. et al. (FEBS Letters 287, 133 - 138, 1991) erwiesen sich die L-Enantiomere als die wirksame Form, im Vergleich zu den Isomerengemischen ist ihre Hemmwirkung um den Faktor 2 erhöht. Die Hemmwirkung der D-Formen liegt um 2 Grössenordnungen niedriger.

Die 2-Naphthylsulfonyl-Schutzgruppe kann durch einen AC-$SO_2$-, Pmc-$SO_2$- Mtr-$SO_2$-, Cm-$SO_2$- oder TIPP-$SO_2$-Rest ersetzt werden. Es werden ebenfalls sehr wirksame Inhibitoren gefunden.

Die in Tabelle 1 nicht mit (L) oder (D) gekennzeichneten Verbindungen sind Racemate und NAPS-F(3AMD) -Pzd(N-COOEt) bedeutet N-a-(2-Naphthylsulfonyl)-(D,L)-3-amidinophenylalanin-4-ethoxycarbonylpiperazid.

Tabelle 1

Hemmung verschiedener Trypsin-ähnlicher Serinproteinasen durch substituierte Piperazide von Nα-geschützten 3-substituierten Phenylalaninen

| $R^1$ | $R^2$ | $R^3$ | Nr | $K_i$, µmol/l Thrombin | Trypsin | Plasmin | Faktor Xa | Faktor XIIa | Plasma Kallikr. | Gland. Kallikr. | tPA |
|---|---|---|---|---|---|---|---|---|---|---|---|
| N A P A P | | | | 0.006 | 0.69 | 30 | 7.9 | 450 | 14 | 93 | 70 |
| NAPS-F(3AMD)-Pzd(N-COOEt) | | | X | 0.67 | 0.022 | 2.5 | 54 | 210 | 30 | > 1000 | 370 |
| AMD | 2-Naph | For | | 0.037 | 0.22 | 6.3 | 19 | 83 | 16 | 440 | > 1000 |
| AMD(L) | 2-Naph | For | | 0.011 | 0.073 | 3.3 | 9.7 | 42 | 3.6 | > 1000 | 120 |
| AMD | 2-Naph | Ac | 1 | 0.023 | 0.14 | 8.4 | 48 | 210 | 28 | > 1000 | > 1000 |
| AMD(L) | 2-Naph | Ac | 2 | 0.012 | 0.044 | 5.4 | 35 | 150 | 15 | > 1000 | 440 |
| AMD | 2-Naph | $SO_2$Me | | 0.0028 | 0.19 | 12 | 22 | 86 | 77 | > 1000 | > 1000 |
| AMD(L) | 2-Naph | $SO_2$Me | 5 | 0.0021 | 0.067 | 9.0 | 19 | 52 | 9.2 | > 1000 | 190 |
| AMD | 2-Naph | $SO_2$Et | | 0.044 | 0.19 | 3.5 | 24 | 212 | 58 | > 1000 | 820 |
| AMD | 2-Naph | $SO_2$-Ph | | 0.041 | 0.33 | 19 | 24 | 120 | 36 | > 1000 | > 1000 |
| AMD | 2-Naph | $SO_2$-Tol | | 0.022 | 0.22 | 15 | 24 | > 1000 | 30 | > 1000 | > 1000 |
| AMD | 2-Naph | $SO_2$-2-Naph | | 0.024 | 0.40 | 11 | 25 | 90 | 22 | > 1000 | > 1000 |
| AMD | 2-Naph | $SO_2$-TMeP | | 0.075 | 0.59 | 31 | 13 | 690 | 1.0 | > 1000 | > 1000 |
| AMD | 2-Naph | $SO_2$-Mtr | | 0.05 | 1.5 | 37 | 25 | 7.6 | 6.9 | > 1000 | > 1000 |
| AMD | 2-Naph | CO-N$(Me)_2$ | | 0.0088 | 0.17 | 6.1 | 24 | 200 | 47 | > 1000 | > 1000 |
| AMD(L) | 2-Naph | CO-N$(Me)_2$ | 6 | 0.004 | 0.097 | 4.9 | 15 | 51 | 7.3 | > 1000 | > 1000 |
| AMD | 2-Naph | CS-N$(Me)_2$ | | 0.013 | 0.049 | 6.2 | 22 | 130 | 28 | > 1000 | > 1000 |
| AMD | 2-Naph | EtOH | | 0.058 | 1.1 | 42 | 35 | > 1000 | 74 | > 1000 | > 1000 |
| AMD(L) | 2-Naph | EtOH | 3 | 0.036 | 0.60 | 21 | 22 | 320 | 25 | > 1000 | 190 |
| AMD | 1-Naph | $SO_2$Me | 11 | 0.026 | 0.32 | 5.0 | 9.7 | > 1000 | 79 | > 1000 | > 1000 |
| AMD | 1-Naph | CO-N$(Me)_2$ | | 0.068 | 0.25 | 4.8 | 5.9 | 72 | 37 | > 1000 | 270 |

Fortsetzung Tabelle 1

Hemmung verschiedener Trypsin-ähnlicher Serinproteinasen durch substituierte Piperazide von Nα-geschützten 3-substituierten Phenylalaninen

| | | | | $K_i$, µmol/l | | | | | | | |
|---|---|---|---|---|---|---|---|---|---|---|---|
| $R^1$ | $R^2$ | $R^3$ | Nr | Thrombin | Trypsin | Plasmin | Faktor Xa | Faktor XIIa | Plasma Kallikr. | Gland. Kallikr. | tPA |
| AMD | Pmc | Ac | | 0.074 | 0.60 | 11.5 | 150 | 420 | 44 | 760 | 670 |
| AMD | Pmc | $SO_2Me$ | | 0.0074 | 0.76 | 30 | 75 | 140 | 140 | › 1000 | › 1000 |
| AMD(L) | Pmc | $SO_2Me$ | 10 | 0.0053 | 0.32 | 10.3 | 37 | › 1000 | 110 | › 1000 | 600 |
| AMD | Pmc | $CO-N(Me)_2$ | | 0.033 | 0.58 | 13 | 26 | › 1000 | 93 | › 1000 | › 1000 |
| AMD | Pmc | EtOH | | 0.059 | 3.3 | 48 | 91 | › 1000 | 180 | › 1000 | › 1000 |
| AMD | Mtr | $SO_2Me$ | | 0.032 | 1.8 | 18 | 120 | 630 | 150 | › 1000 | › 1000 |
| AMD(L) | Mtr | $SO_2Me$ | 9 | 0.020 | 0.86 | 8.2 | 48 | › 1000 | 170 | › 1000 | 600 |
| AMD(L) | (-)Cm | $SO_2Me$ | 7 | 0.012 | 0.22 | 14 | 1.1 | 61 | 25 | › 1000 | 20 |
| AMD(L) | (+)Cm | $SO_2Me$ | 8 | 0.031 | 0.44 | 29 | 6.7 | 70 | 130 | › 1000 | 82 |
| AMD | TIPP | Ac | | 0.098 | 0.22 | 3.0 | 4.0 | 47 | 7.3 | 200 | 17 |
| AMD | AC | Ac | 4 | 0.014 | 0.026 | 8.6 | 4.1 | 52 | 7.0 | › 1000 | › 1000 |
| AMD | AC | For | | 0.031 | 0.033 | 13 | 1.7 | 7.2 | 11 | › 1000 | 84 |
| AMD | 2-Naph | 2-Pyl | | 0.52 | 0.035 | 3.3 | 12 | 84 | 19 | › 1000 | › 1000 |
| AMD | 2-Naph | 2-Pym | | 1.8 | 0.061 | 3.3 | 14 | 100 | 19 | › 1000 | 320 |
| AMD | 2-Naph | $COCH(Me)_2$ | | 0.26 | 0.063 | 9.3 | 84 | › 1000 | 50 | › 1000 | 440 |
| AMD | 2-Naph | $COCH_2OMe$ | | 1.3 | 0.066 | 6.2 | 16 | 74 | 16 | › 1000 | › 1000 |
| AMD | 2-Naph | $CO(CH_2)_4CH_3$ | | 6.5 | 0.064 | 2.6 | 19 | 270 | 23 | › 1000 | › 1000 |
| AMD | 2-Naph | CO-cPr | | 0.14 | 0.044 | 5.1 | 45 | 540 | 22 | › 1000 | 170 |
| AMD | 2-Naph | CO-cBu | | 2.5 | 0.026 | 7.6 | 58 | › 1000 | 56 | › 1000 | › 1000 |
| AMD | 2-Naph | CO-cHex | | 36 | 0.072 | 10.2 | 58 | 350 | 79 | › 1000 | › 1000 |
| AMD | 2-Naph | $CO-CH_2-$ 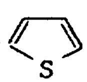 | | 2.5 | 0.081 | 2.7 | 16 | 72 | 19 | › 1000 | 170 |
| Ame | 2-Naph | 2-Pyl | | 24 | 0.53 | 26 | 130 | › 1000 | 94 | › 1000 | › 1000 |

Tabelle 2 stellt eine Übersicht aller synthetisierten und in vitro geprüften Verbindungen, die nicht in Tabelle 1 enthalten sind, dar.

<u>Tabelle 2</u>

Zusammenstellung der synthetisierten Verbindungen der allgemeinen Formel I, die nicht in Tabelle 1 aufgeführt sind

| $R^1$ | $R^2$ | $R^3$ | $R^1$ | $R^2$ | $R^3$ | $R^1$ | $R^2$ | $R^3$ |
|---|---|---|---|---|---|---|---|---|
| AMD(D) | 2-Naph | Ac | AMD | 2-Naph | $SO_2CF_3$ | AMe | 2-Naph | $SO_2Et$ |
| AMD(D) | 2-Naph | EtOH | AMD | 2-Naph | $SO_2N(Me)_2$ | AMe | 2-Naph | $SO_2CF_3$ |
| AMD(D) | 2-Naph | For | AMD | 2-Naph | $SO_2$-1-Naph | AMe | 2-Naph | $SO_2N(Me)_2$ |
| AMD | Cm | Ac | AMD | 2-Naph | TIPP | AMe | 2-Naph | $SO_2$-Ph |
| AMD(L) | Tol | Ac | AMD | 2-Naph | $SO_2$-Pmc | AMe | 2-Naph | $SO_2$-Tol |
| AMD | 2-Naph | AcONHiPr | AMD | 2-Naph | $SO_2$-(-)Cm | AMe | 2-Naph | TIPP |
| AMD | 2-Naph | $SO_2CH(Me)_2$ | AMD | 2-Naph | Bzl | AMe | 2-Naph | $SO_2$-2-Naph |
| AMD | 2-Naph | EtOEtOH | AMD | Tol | Ac | AMe | 2-Naph | 2-Pym |
| AMD | 2-Naph | H | AMD | TIPP | EtOH | AMe | Tol | Ac |
| AMD | 2-Naph | $CH_2CO$-N | AMD | TIPP | $CON(Me)_2$ | AMe | Mtr | Ac |
| | | | AMD | TIPP | $SO_2Me$ | Ame | Mtr | $SO_2Me$ |
| AMD | 2-Naph | COEt | AMD | TIPP | $SO_2$-2-Naph | AMe | Pmc | Ac |
| AMD | 2-Naph | $COCH_2CH(Ph)_2$ | AMD | Mtr | Ac | AMe | Pmc | EtOH |
| AMD | 2-Naph | $COC(Me)_3$ | AMD | Mtr | EtOH | AMe | Pmc | $SO_2Me$ |
| AMD | 2-Naph | CO-Ph | AMD | Mtr | $CON(Me)_2$ | AMe | Pmc | 2-Pyl |
| AMD | 2-Naph | CO-Ph-OMe(p) | AMD | Mtr | $SO_2$-2-Naph | AMe | AC | Ac |
| AMD | 2-Naph | CO-(2)-Naph | AMD | 1-Naph | Ac | AMe | 2-Naph | For |
| AMD | 2-Naph | CO-$CH_2$-$NH_2$ | AMD | 1-Naph | EtOH | AMe | 2-Naph | Ac |
| AMD | 2-Naph | CO-$CH_2NHCOCOMe$ | AMD(D) | Pmc | $SO_2Me$ | AMe | 2-Naph | H |
| AMD | 2-Naph | CO-$CH_2NHBoc$ | AMD | Pmc | $SO_2$-2-Naph | AMe | 2-Naph | COEt |
| AMD | 2-Naph | COCOOMe | AMD | Pmc | 2-Pyl | AMe | 2-Naph | $CON(Me)_2$ |
| AMD | 2-Naph | $CH_2COOMe$ | AMD | Cm | Ac | AMe | 2-Maph | $SO_2Me$ |
| | | | AMD | 2-Naph | CO-N 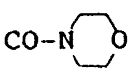 | AMe | 2-Naph | $CH_2CO$-N 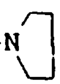 |
| AMD(D) | 2-Naph | CO-$N(Me)_2$ | AMD | 2-Naph | $CH_2$- 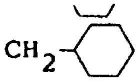 | | | |
| AMD | 2-Naph | CO-$N(Et)_2$ | | | | | | |
| AMD(D) | 2-Naph | $SO_2Me$ | | | | | | |

In Tabelle 3 - 5 sind die Ergebnisse von Untersuchungen zur Pharmakokinetik von repräsentativen, erfindungsgemässen Verbindungen und als Vergleich dazu die Werte mit NAPAP zusammengestellt, Tabelle 6 enthält die Ergebnisse ausgewählter Verbindungen nach intraduodenaler Applikation. Die zu prüfenden Verbindungen wurden intravenös (Tabelle 3), peroral (Tabelle 4), rektal (Tabelle 5) bzw. intraduodenal (Tabelle 6) an Ratten verabreicht. Nach der Verabreichung wurde den Versuchstieren in Zeitabständen von 2 bis maximal 360 Minuten Blutproben entnommen, in welchen der Blutspiegel der zu prüfenden Verbindungen mittels HPLC bestimmt wurde.

Tabelle 3

| Konzentration (ng/ml) ausgewählter Verbindungen im Plasma von Ratten nach intravenöser Verabreichung von 1 mg/kg | | | | | | | |
|---|---|---|---|---|---|---|---|
| | Verbindung | | | | | | |
| Zeit (min) | NAPAP | Nr. X | Nr. 1 | Nr. 2 | Nr. 3 | Nr. 5 | Nr. 6 |
| 5 | 1150 | 812 | 1058 | 780 | 2210 | 879 | 823 |
| 15 | 428 | 169 | 394 | 224 | 145 | 367 | 289 |
| 30 | 376 | 40 | 150 | 95 | 313 | 151 | 194 |
| 60 | 192 | 8 | 89 | 38 | 112 | 50 | 141 |
| 120 | 84 | 0 | 74 | 12 | 0 | 0 | 86 |
| 180 | 106 | 0 | 51 | 10 | 0 | 0 | 70 |

Tabelle 4

| Konzentration (ng/ml) ausgewählter Verbindungen im Plasma von Ratten nach oraler Verabreichung von 50 mg/kg | | | | |
|---|---|---|---|---|
| | Verbindung | | | |
| Zeit (min) | NAPAP | Nr. X | Nr. 1 | Nr. 2 |
| 30 | 0 | 33 | 87 | 1197 |
| 60 | 0 | 10 | 63 | 873 |
| 120 | 0 | 0 | 57 | 333 |
| 180 | 0 | 0 | 69 | 124 |
| 240 | 0 | 0 | 91 | 44 |
| 300 | 0 | 0 | 61 | 46 |

Tabelle 5

| Konzentration (ng/ml) ausgewählter Verbindungen im Plasma von Ratten nach rektaler Verabreichung | | | | | | | | |
|---|---|---|---|---|---|---|---|---|
| | Verbindung | | | | | | | |
| Zeit (min) | Dosis | NAPAP | Nr.X | Nr.1 | Nr.2 | Nr.3 | Nr.5* | Nr.6 |
| | mg/kg | 100 | 100 | 100 | 100 | 20 | 20 | 20 |
| 30 | | 18 | 3255 | 31406 | 32448 | 7260 | 1650 | 1179 |
| 60 | | 0 | 2371 | 17862 | 23132 | 3800 | 958 | 449 |
| 120 | | 0 | 1804 | 3776 | 5302 | 1740 | 299 | 850 |
| 180 | | 0 | 898 | 1075 | 2696 | 814 | 192 | 272 |
| 240 | | 0 | 626 | 438 | 1263 | 215 | | 46 |
| 300 | | 0 | 592 | 220 | 810 | 61 | | |
| 360 | | 0 | 448 | 158 | 508 | 34 | | |

* = freie Base der Verbindung

Tabelle 6

| Konzentration (ng/ml) ausgewählter Verbindungen im Plasma von Ratten nach intraduodenaler Verabreichung von 100 mg/kg | | | | | | |
|---|---|---|---|---|---|---|
| | Verbindung | | | | | |
| Zeit (min) | Nr.2 | Nr.3 | Nr.3* | Nr.5 | Nr.5* | Nr.6 |
| 30 | 21691 | 3824 | 2262 | 8744 | 1576 | 20460 |
| 60 | 15152 | 2172 | 795 | 9314 | 1900 | 6495 |
| 120 | 4275 | 1457 | 361 | 8612 | 1763 | 3284 |
| 180 | 2598 | 945 | 228 | | 297 | 2064 |
| 240 | 2185 | 1277 | 144 | | 530 | 1500 |
| 300 | 2195 | 1400 | 276 | | 137 | 1203 |
| 360 | 2847 | | | | 119 | 1437 |

* = freie Base der Verbindung

Die geprüften Derivate zeigen nicht nur im Vergleich zu NAPAP ein verbessertes pharmakokinetisches Verhalten, sondern auch zu dem früher beschriebenen Piperazid X. Zwar werden die erfindungsgemässen Verbindungen 1, 2, 3, 5 und 6 nach intravenöser Gabe mit vergleichbarer Geschwindigkeit eliminiert und nach oraler Applikation nur in geringerem Masse resorbiert, nach rektaler Verabreichung werden aber z.T. sehr hohe, 1 bis 2 Stunden andauernde Blutspiegel gefunden. Nach rektaler Gabe kann NAPAP nicht im Plasma nachgewiesen werden, während einige der beispielhaft geprüften erfindungsmässigen Verbindungen ausserordentlich hohe Konzentrationen erreichen. Verbindung 1, 2 und 3 sind bis zu 6 Stunden im Plasma nachzuweisen. Die nach rektaler Applikation erreichten Plasmaspiegel liegen deutlich über denen des früher beschriebenen Piperazids X. Auch die nach intraduodenaler Applikation gemessenen Plasmakonzentrationen, z.T. über mehrere Stunden, sind beträchtlich.

In vitro sind eine Reihe von repräsentativen erfindungsgemässen Verbindungen gerinnungshemmend ausserordentlich wirksam. In allen Fällen wurde die Thrombinzeit (TT) am effektivsten verlängert. Dies entspricht der Selektivität dieser Inhibitoren, die unter den Gerinnungsfaktoren Thrombin am stärksten hemmen. Beispielhaft ist das für die Verbindungen 1 bis 11 in Tabelle 7 gezeigt. Eine Verlängerung der aktivierten partiellen Thromboplastinzeit (aPTT), bei der neben Thrombin auch die an der Frühphase der Gerinnung beteiligten Enzyme zum Tragen kommen, wird durch höhere Konzentrationen der Inhibitoren erreicht. Das gilt auch für die Beeinflussung der Prothrombinzeit (PT), die den extrinsischen Gerinnungsweg repräsentiert. In Tab. 7 sind die Konzentrationen aufgeführt, die zur Verdopplung der Gerinnungszeiten notwendig sind. Für die wirksamen Thrombinhemmstoffe 1, 2, 4, 5, 6 und 7 liegt der Wert unter $10^{-7}$ mol/ für die Verlängerung der TT, bei 1 μmol/l für die Verlängerung von aPTT und PT. Die vergleichsweise geprüften wirksamen Hemmstoffe NAPAP und Verbindung X sind entsprechend ihrem $K_i$-Wert effektiv.

Im Plasma sind die Hemmstoffe vom Piperazin-Typ (absolut) stabil. Bei Inkubation im Human-Plasma bei 37°C trat über 5 h keine Veränderung der Hemmwirkung auf.

Tabelle 7

| Gerinnungshemmung im menschlichen Plasma durch ausgewählte Verbindungen | | | | |
|---|---|---|---|---|
| | Thrombin-Hemmung | Konzentr. [μmol/l] zur Verdopplung von | | |
| Nr. | $K_i$, μmol/l | Thrombinzeit | aPTT | Prothrombinzeit |
| NAPAP | 0.006 | 0.048 | 0.50 | 1.0 |
| X | 0.67 | 4.1 | 20 | 45 |
| 1 | 0.023 | 0.095 | 0.90 | 2.5 |
| 2 | 0.012 | 0.055 | 0.36 | 0.90 |
| 3 | 0.036 | 0.14 | 0.65 | 1.3 |
| 4 | 0.014 | 0.085 | 1.2 | 2.0 |
| 5 | 0.0021 | 0.034 | 0.26 | 0.39 |
| 6 | 0.004 | 0.042 | 0.3 | 0.65 |
| 7 | 0.012 | 0.075 | 0.55 | 1.0 |
| 8 | 0.031 | 0.13 | 1.2 | 2.0 |
| 9 | 0.020 | 0.12 | 0.57 | 1.1 |

Tabelle 7 (fortgesetzt)

| Gerinnungshemmung im menschlichen Plasma durch ausgewählte Verbindungen | | | | |
|---|---|---|---|---|
| | Thrombin-Hemmung | Konzentr. [μmol/l] zur Verdopplung von | | |
| Nr. | $K_i$, μmol/l | Thrombinzeit | aPTT | Prothrombinzeit |
| 10 | 0.0053 | 0.10 | 0.44 | 0.8 |
| 11 | 0.026 | 0.22 | 1.8 | 3.1 |

Die antikoagulierende Wirkung der Verbindungen lässt sich auch in vivo nachweisen. Nach rektaler Verabreichung der zu prüfenden Verbindungen wurde im Plasma der Versuchstiere der gerinnungshemmende Effekt bestimmt. Beispielhaft ist das für die Verbindungen 2, 3, 5 und 6 in Tabelle 8 gezeigt. Ganz entsprechend dem mittels HPLC bestimmten Konzentrationsverlauf im Plasma ist die Antithrombinwirkung im Gerinnungstest nachzuweisen.

Tabelle 8

| Gerinnungshemmung im Plasma der Ratte nach rektaler Applikation der Verbindungen 2, 3, 5 und 6 | | | |
|---|---|---|---|
| Zeit (min) | Konzentration im Plasma (ng/ml) | Gerinnungszeit (sec) | |
| | | Thrombinzeit | aPTT |
| | Verbindung 2 | 20 mg/kg | |
| 0 | 0 | 64 | 28 |
| 30 | 5440 | 300 | 120 |
| 60 | 2090 | > 300 | 87 |
| 120 | 812 | > 300 | 59 |
| 180 | 660 | > 300 | 50 |
| | Verbindung 2 | 5 mg/kg | |
| 0 | 0 | 32 | 22.5 |
| 30 | 296 | > 300 | 37 |
| 60 | 160 | 224 | 33 |
| 120 | 60 | 176 | 30 |
| 180 | 40 | 107 | 26.4 |
| | Verbindung 3 | 5 mg/kg | |
| 0 | 0 | 45 | 23.5 |
| 30 | 897 | > 300 | 39.8 |
| 60 | 462 | 298 | 32.8 |
| 120 | 355 | 165 | 27.4 |
| 180 | 49 | 114 | 26.0 |
| | Verbindung 5 | 20 mg/kg | |
| 0 | 0 | 153 | 22 |
| 30 | 1572 | > 300 | 65 |
| 60 | 983 | > 300 | 62.5 |
| 120 | 380 | > 300 | 36 |
| 180 | 195 | > 300 | 29.7 |
| | Verbindung 5 | 5 mg/kg | |
| 0 | 0 | 51.5 | 21.2 |
| 30 | 168 | > 300 | 34.5 |
| 60 | 65 | 255 | 30.5 |
| 120 | 82 | 137.5 | 27.5 |
| 180 | 10 | 95 | 25.3 |

Tabelle 8 (fortgesetzt)

| Gerinnungshemmung im Plasma der Ratte nach rektaler Applikation der Verbindungen 2, 3, 5 und 6 | | | |
|---|---|---|---|
| Zeit (min) | Konzentration im Plasma (ng/ml) | Gerinnungszeit (sec) | |
| | Verbindung 6 | 5 mg/kg | |
| 0 | 0 | 55.3 | 22.3 |
| 30 | 648 | > 300 | 34.5 |
| 60 | 362 | > 300 | 31.5 |
| 120 | 157 | > 300 | 28.4 |
| 180 | 83 | 213 | 26 |

Die erfindungsgemässen Verbindungen werden nach rektaler Applikation in einer Grössenordnung resorbiert, die zu antithrombotisch wirksamen Plasmaspiegeln führt. Das wurde am Modell der Stase-induzierten venösen Thrombose bei der Ratte nach Wessler et al. gezeigt. Dazu wurden die in den Jugularvenensegmenten durch Serum ausgelösten Thromben anhand eines Scores (0 = flüssiges Blut, 1 = ein oder mehrere kleine Thromben, 2 = Gefässsegment nicht vollständig verschlossen, 3 = Gefässsegment vollständig verschlossen) makroskopisch bewertet. Die antithrombotische Wirkung ist deutlich dosis-abhängig. Bei einer Dosis von 100 bzw. 20 mg/kg wird die Thrombusbildung vollständig unterdrückt, und noch bei der niedrigen Dosierung von 5 mg/kg wird ein Gefässverschluss weitgehend verhindert resp. die Thrombusbildung weitgehend zurückgedrängt.

Tabelle 9

| Dosisabhängige antithrombotische Wirksamkeit nach rektaler Applikation von Verbindung 2 am Modell der Stase-induzierten venösen Thrombose bei der Ratte | | | | | | | | |
|---|---|---|---|---|---|---|---|---|
| | | Plasma-Spiegel (ng/ml) | | Thrombus-Score | | | | relativer Thrombus-Score |
| Dosis (mg/kg) | n | 30 min | 60 min | 0 | 1 | 2 | 3 | |
| 0 | 5 | 0 | 0 | 0 | 0 | 1 | 9 | 1 |
| 100 | 3 | 28698 | 16118 | 6 | 0 | 0 | 0 | 0 |
| 20 | 4 | 1617 | 998 | 6 | 1 | 0 | 0 | 0.05 |
| 5 | 5 | 228 | 160 | 0 | 8 | 2 | 0 | 0.41 |
| n = Anzahl der Versuchstiere mit in der Regel je 2 ausgewerteten Venensegmenten. | | | | | | | | |

Der relative Thrombus-Score ist der Quotient aus der mittleren Thrombus-Grösse der behandelten Gruppe (z.B. beträgt der mittlere Score bei 5 mg/kg 1,2) und der mittleren Thrombus-Grösse der Kontrollgruppe (2,9).

Unter Verwendung geeigneter Hilfs- bzw. Trägerstoffe können die nach den erfindungsgemässen Verfahren hergestellten Piperazide als solche oder als Salze physiologisch verträglicher anorganischer oder organischer Säuren in geeignete Applikationsformen übergeführt werden, wobei entsprechend dem pharmakologischen Verhalten Tabletten, Dragees, Suppositorien und Lösungen von besonderer Bedeutung sind.

Die Dosierung richtet sich vor allem nach der Antithrombinaktivität, den erreichbaren Blutspiegelwerten bei entsprechender Applikationsform, der Bioverfügbarkeit sowie der Allgemeinkonstitution des Patienten, wobei mit Dosierungen zwischen 0.5 und 50 mg/kg eine ausreichende antithrombotische Aktivität erreicht werden kann.

Anhand von Nα-(2-Naphthylsulfonyl)-(L)-3-amidinophenylalanin-4-acetylpiperazid-hydrochlorid (Verbindung 2) soll die Überführung in 3 pharmazeutische Applikationsformen beispielhaft gezeigt werden.

Beispiel 1

Tabletten mit 20 mg der Verbindung 2 als Wirkstoff, überzogen mit einem magensaftresistenten Schutzlack.

Zusammensetzung:

Tablettenkern: 20 mg Wirkstoff, 96 mg Lactose, 3 mg Talcum und 1 mg Magnesiumstearat.
Lack: 23.92 mg Eudragit S 12.5P (Röhm Pharma, Darmstadt), 0.266 mg Dibutylphthalat, 0.744 mg Talcum, 23.92 mg

Aceton/Ethanol 1+1.

Herstellungsverfahren:

Der mit den Hilfsstoffen vermischte Wirkstoff wird durch ein Sieb der Maschenweite 0.5 mm gedrückt und in trockener Form zu ovalen Tablettenkernen gepresst. Anschliessend wird in einem Wirbelschichtgranulator der Schutzlack aufgesprüht und die lackierten Kerne danach getrocknet.

Beispiel 2

Suppositorien (Zäpfchen) mit 10 mg der Verbindung 2 als Wirkstoff.

Zusammensetzung:

1 Zäpfchen enthält 10 mg Wirkstoff und 1 g Witepsol W45 als Grundlage.

Herstellungsvorschrift für 10 Zäpfchen:

100 mg des feinst gepulverten Wirkstoffs werden mit 1 g der verflüssigten Grundlage verrieben. Die Verreibung wird mit dem Rest der verflüssigten Grundlage anteilweise gemischt und bis zur gleichmässigen Beschaffenheit bearbeitet. Nahe der Grenze der Giessbarkeit wird die Mischung in eine geeignete Form gegossen und bis zum Erkalten stehengelassen.

Beispiel 3

Injektions- bzw. Infusionslösung mit 2.5 mg/ml der Verbindung 2 als Wirkstoff.

Herstellungsverfahren:

0.25 g Wirkstoff werden in 100 ml Aqua ad injectionem gelöst, die Lösung filtriert und gegebenenfalls in Ampullen zu je 2 ml abgefüllt. Die mit der Wirkstofflösung gefüllten und verschlossenen Gefässe (Infusicnsflaschen, Ampullen) werden der Dampfsterilisation bei 121 bis 124°C unterzogen.

## Patentansprüche

1. D,L-, L- oder D-Phenylalanin-piperazide der Formel

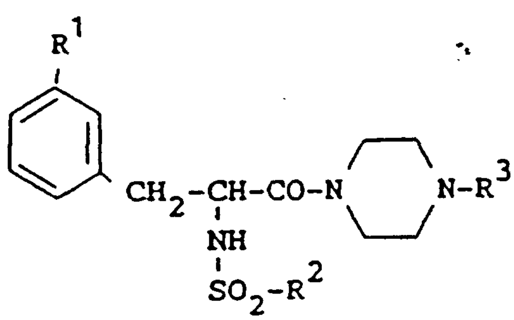

I

in denen $R^1$ eine basische Gruppe der Formel

(a) $HN{=}C{-}NH_2$ oder (b) $-CH_2-NH_2$

Amidino    Aminomethyl

darstellt;
$R^2$ Phenyl, 4-Methyl-phenyl, 2,4,6-Trimethyl- bzw. -Triisopropyl-phenyl, 4-Methoxy-2,3,6-trimethyl-phenyl, 2,2-Dimethyl-6-methoxy- bzw. 2,2,5,7,8-Pentamethyl-chromanyl, Anthrachinonyl, 1- bzw. 2-Naphthyl, Chinolyl bzw. Isochinolyl oder einen Campherrest darstellt; und
$R^3$ einen Acylrest der Formel -COX darstellt, wobei X Wasserstoff, $C_{1-3}$-Alkyl oder $C_{3-10}$-Cycloalkyl darstellt; oder
einen Benzylrest darstellt, in dem der aromatische Rest gegebenenfalls mit einem Halogenatom oder einer $C_{1-3}$-Alkyl-, $C_{1-3}$-Alkoxy-, Hydroxy- oder Nitrogruppe substituiert ist; oder
einen Carbonsäureamidrest der Formel $-CONR^iR^{ii}$, einen Thiocarbonsäureamidrest der Formel $-CSNR^iR^{ii}$ oder einen Essigsäureamidrest der Formel $-CH_2-CONR^iR^{ii}$ darstellt, wobei $R^i = R^{ii} = H$; $R^i = R^{ii} = C_{1-3}$-Alkyl; $R^i = H$, $R^{ii} = C_{1-3}$-Alkyl; $R^i = H$, $R^{ii}$ = Phenyl; oder $R^i$ und $R^{ii}$ zusammen mit dem Stickstoffatom einen 5- bis 7-gliedrigen Ring bilden, welcher ein weiteres Heteroatom enthalten kann; oder
einen Rest der Formel $-SO_2-Y$ darstellt, in dem Y $C_{1-3}$-Alkyl, Trifluor-methyl, Trichlor-methyl, Phenyl, 4-Methyl-phenyl, 2,4,6-Trimethyl- bzw. -Triisopropyl-phenyl, 4-Methoxy-2,3,6-trimethyl-phenyl, 2,2-Dimethyl-6-methoxy- bzw. 2,2,5,7,8-Pentamethyl-chromanyl, Anthrachinonyl, Naphthyl, Chinolyl oder $-NR^{iii}R^{iv}$ bedeutet, wobei $R^{iii}$ und $R^{iv}$ jeweils H und/oder gleiches oder ungleiches $C_{1-3}$-Alkyl sind; oder
einen $C_{5-8}$-Cycloalkylring darstellt, der gegebenenfalls mit einer Hydroxyl- oder Oxogruppe substituiert sein kann; oder
einen Pyridyl-, Pyrimidinyl- oder N-Methylpiperidylrest darstellt; oder
einen funktionalisierten Alkylrest der Formel $-(CH_2)_n-X'$ darstellt, wobei die Alkylkette unverzweigt oder verzweigt ist, n 1 bis 8 bedeutet und der funktionelle Rest X'
eine Hydroxygruppe darstellt, deren H-Atom gegebenenfalls durch $C_{1-3}$-Alkyl, Benzyl, Phenyl, 4-Methyl-phenyl, 2,4,6-Trimethyl- bzw. -Triisopropyl-phenyl, 4-Methoxy-2,3,6-trimethyl-phenyl, Anthrachinonyl, Naphthyl, Hydroxy-$C_{1-3}$-Alkyl oder durch eine Acylgruppe der obigen Formel -COX ersetzt ist, oder
ein Halogenatom bedeutet, oder
eine tertiäre Aminogruppe der Formel $-NR^vR^{vi}$ darstellt, wobei $R^v$ und $R^{vi}$ entweder gleiche $C_{1-3}$-Alkylgruppen sind oder zusammen mit dem Stickstoffatom einen 5- bis 7-gliedrigen Ring bilden, wobei gegebenenfalls ein oder zwei weitere Ringe annelliert ist/sind, oder
eine Acylaminomalonsäurediestergruppe der Formel

$$X-CO-HN-C(COO-C_{1-3}-Alkyl)_2,$$

worin X obige Bedeutung besitzt, eine Gruppe der Formel

$$X-CO-HN-CH-COOH,$$

worin X obige Bedeutung besitzt, oder eine Gruppe der Formel

$$H_2N-CH-COOH$$

darstellt;
und deren Salze mit Mineralsäuren oder organischen Säuren.

**2.** Phenylalanin-Piperazide gemäss Anspruch 1, worin Y im Rest der Formel $-SO_2-Y$ Methyl, Trifluormethyl oder Trichlormethyl ist.

**3.** Phenylalanin-Piperazide gemäss Anspruch 1 oder 2, worin $R^1$ eine Amidinogruppe der Formel (a) darstellt und/ oder $R^2$ 2-Naphthyl, Anthrachinonyl, 2,4,6-Triisopropylphenyl oder 2,2,5,7,8-Pentamethyl-chromanyl bedeutet und/oder $R^3$ einen Acylrest der Formel -COX, einen funktionalisierten Alkylrest der Formel $-(CH_2)_n-X'$, einen Rest der Formel $-SO_2-Y$, einen Carbonsäureamidrest der Formel $-CONR^iR^{ii}$ darstellt oder 2-Pyridyl oder 2-Pyrimidinyl bedeutet.

**4.** Phenylalanin-Piperazide gemäss Anspruch 3, worin $R^3$ Formyl, Acetyl oder 2-Hydroxyethyl bedeutet.

**5.** Verfahren zur Herstellung von Phenylalanin-piperaziden gemäss einem der Ansprüche 1-4, worin R1 eine Amidinogruppe der Formel (a) darstellt, **dadurch gekennzeichnet, dass** man einen (D, L)-3-Cyanphenylalanin-alkylester der Formel II

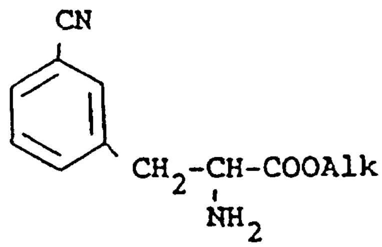

mit einem Sulfochlorid der Formel $R^2-SO_2Cl$, in der $R^2$ die in Anspruch 1 definierte Bedeutung aufweist, zu einer racemischen Verbindung der Formel IV

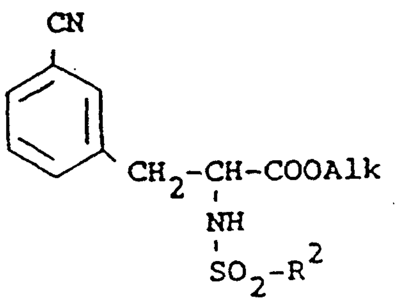

umsetzt, aus welcher durch Hydrolyse das Racemat des sulfonylierten Cyanphenylalanins der Formel V

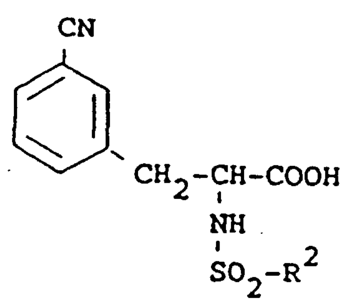

erhalten wird, oder die Verbindung der Formel IV durch enzymatische Esterhydrolyse mittels Chymotrypsin in die L-konfigurierte Aminosäure der Formel V überführt und den dabei anfallenden Aminocarbonsäure-alkylester mit D-Konfiguration der Formel IV durch Hydrolyse in die D-konfigurierten Aminocarbonsäuren der Formel V überführt, die Verbindung der Formel V. durch Kupplung mit einem Piperazin-Derivat der Formel VII

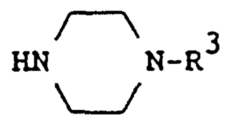

in die (D,L)-, D- oder L-Cyanverbindung mit Piperazid-Struktur der Formel VI

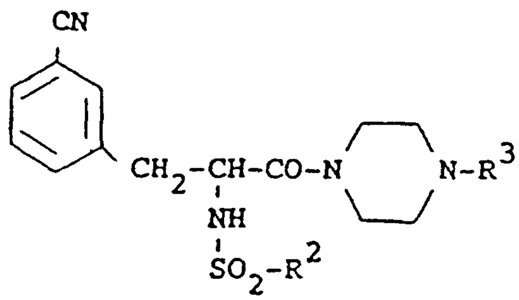

überführt, durch Addition von $H_2S$ an den Cyanrest das Thioamid der Formel VIII

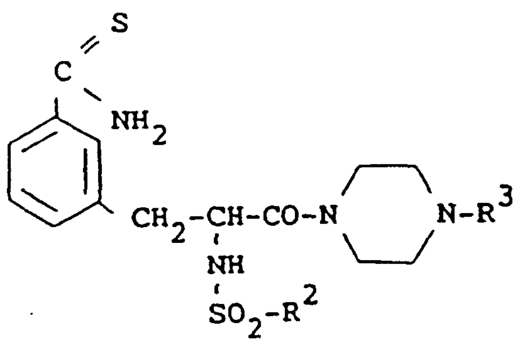

herstellt, durch Umsetzung mit einem Alkylhalogenid ein Thioimidsäureesterhalogenid der Formel IX

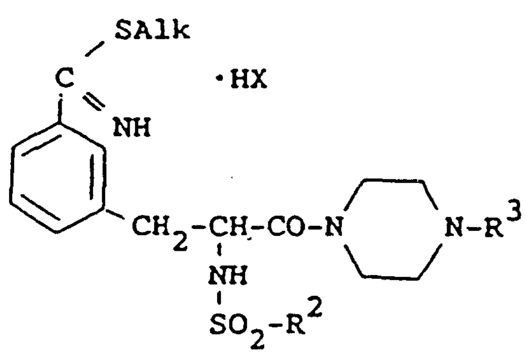

herstellt, oder aus der Cyanverbindung mit Piperazid-Struktur der Formel VI ein Imidsäureesterhalogenide der Formel X

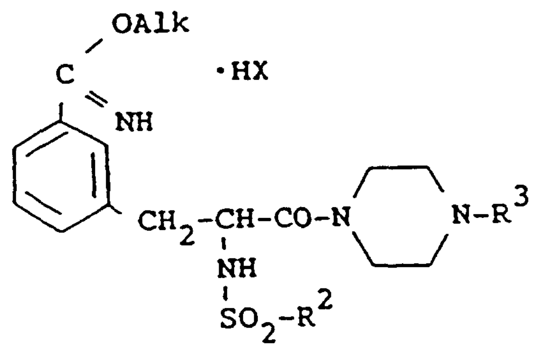

herstellt, und die Verbindung der Formel IX mit Ammoniumacetat oder die Verbindung der Formel X in alkoholischer Ammoniaklösung zu einer Verbindung der Formel XI

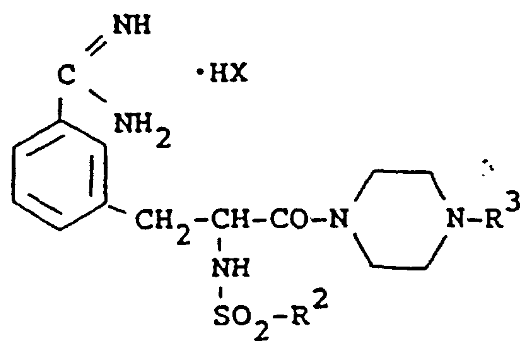

in der X Halogen, vorzugsweise Chlor bedeutet, umsetzt.

**6.** Variante des Verfahrens gemäss Anspruch 5, **dadurch gekennzeichnet, dass** man zur Herstellung der als Zwischenprodukt anfallenden (D,L)-, D- oder L-Cyanverbindung mit Piperazid-Struktur der Formel VI ein (D,L), L- oder D-3-Cyanphenylalanin durch Einführen einer Boc-Gruppe an der Aminogruppe schützt, die erhaltene Carbonsäure entsprechend der Formel V, jedoch mit der Boc-Gruppe statt der $SO_2$-$R^2$-Gruppe, mit einem Piperazin-Derivat der Formel VII in die Boc-geschützte (D,L)-, D- oder L-Cyanverbindung mit Piperazid-Struktur entsprechend der Formel VI, jedoch mit der Boc-Gruppe statt der $SO_2$-$R^2$-Gruppe, umsetzt und diese Verbindung nach saurer Abspaltung der Boc-Gruppe und Umsetzung mit einem Sulfochlorid der Formel $R^2$-$SO_2Cl$ in die Verbindung der Formel VI überführt.

**7.** Verfahren zur Herstellung von Phenylalanin-piperaziden gemäss einem der Ansprüche 1-4, worin der $R^1$ Aminomethyl der Formel (b) bedeutet, **dadurch gekennzeichnet, dass** man eine Cyanverbindung der in Anspruch 5 definierten Formel VI hydriert.

**8.** Verfahren gemäss Anspruch 7, **dadurch gekennzeichnet, dass** man die Verbindung der Formel VI katalytisch hydriert.

**9.** Verwendung der Phenylalanin-piperazide gemäss einem der Ansprüche 1-4 in Salzform oder als freie Basen zur Herstellung von subkutan oder intravenös, insbesondere aber oral, rektal oder intraduodenal, verabreichbaren antithrombotisch wirksamen Arzneimitteln.

**10.** Subkutan oder intravenös, insbesondere aber oral, rektal oder duodenal, verabreichbares antithrombotisch wirksames Arzneimittel, **gekennzeichnet durch** eine wirksame Menge mindestens eines Phenylalanin-piperazids gemäss einem der Ansprüche 1-4 und geeignete Hilfsstoffe.

**11.** Antithrombotisch wirksames Arzneimittel gemäss Anspruch 10, in Form von Tabletten, Dragees, Kapseln, Pellets, Suppositorien, LÖsungen, Injektionen oder transdermalen Systemen, wie Pflaster.

## Claims

**1.** D,L-, L- or D-phenylalanine piperazides of formula

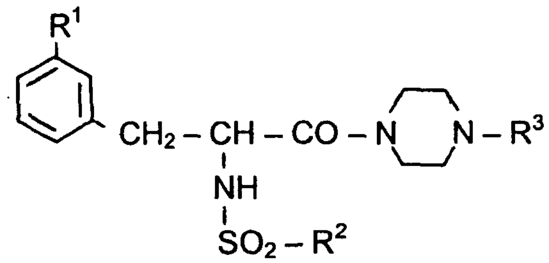

I

wherein $R^1$ represents a basic group of formula

(a) $HN=C-NH_2$ amidino    or    (b) $-CH_2-NH_2$ aminomethyl

$R^2$ represents phenyl, 4-methylphenyl, 2,4,6-trimethyl- or -triisopropylphenyl, 4-methoxy-2,3,6-trimethylphenyl, 2,2-dimethyl-6-methoxy- or 2,2,5,7,8-pentamethyl-chromanyl, anthraquinonyl, 1- or 2-naphthyl, quinolyl or isoquinolyl or a camphor residue, respectively; and

$R^3$ represents an acyl residue of formula —COX, wherein X means hydrogen, alkyl $C_{1-3}$ or cycloalkyl $C_{3-10}$; or

a benzyl residue wherein the aromatic residue may be substituted with a halogen atom or an alkyl $C_{1-3}$, alkoxy $C_{1-3}$, hydroxy or nitro group; or

a carboxamide residue of formula —$CONR^iR^{ii}$, a thiocarboxamide residue of formula -$CSNR^iR^{ii}$ or an ethylamide residue of formula —$CH_2$-$CONR^iR^{ii}$ in which $R^i = R^{ii}$ = H; $R^i = R^{ii}$ = alkyl $C_{1-3}$; $R^i$ = H, $R^{ii}$ = alkyl $C_{1-3}$; $R^i$ = H, $R^{ii}$ = phenyl; or $R^i$ and $R^{ii}$ together with the nitrogen atom form a 5- to 7-branched ring which may contain a further hetero atom; or

a residue of formula —$SO_2$-Y in which Y means alkyl $C_{1-3}$, trifluoromethyl, trichloromethyl, phenyl, 4-methylphenyl, 2,4,6-trimethyl- or —triisopropylphenyl, 4-methoxy-2,3,6-trimethylphenyl, 2,2-dimethyl-6-methoxy- or 2,2,5,7,8-pentamethylchromanyl, anthraquinonyl, naphthyl, quinolyl or —$NR^{iii}R^{iv}$, respectively, in which $R^{iii}$ and $R^{iv}$ = H and/or are equal or unequal to alkyl $C_{1-3}$; or

a cycloaliphatic ring $C_{5-8}$ which may be substituted with a hydroxyl or oxo group; or

a pyridyl, pyrimidinyl or N-methylpiperidyl residue; or

a functionalized alkyl residue of formula —$(CH_2)_n$-X' wherein the alkyl chain is unbranched or branched, n = 1 to 8 and the functional residue X' represents

a hydroxy group the H atom of which may be substituted with alkyl $C_{1-3}$, benzyl, phenyl, 4-methylphenyl, 2,4,6-trimethyl- or —triisopropylphenyl, 4-methoxy-2,3,6-trimethylphenyl, anthraquinonyl, naphthyl, hydroxy alkyl $C_{1-3}$ or an acyl group of above formula —COX, or

a halogen atom, or

a tertiary amino group of formula —$NR^vR^{vi}$ wherein $R^v$ and $R^{vi}$ are either same alkyl $C_{1-3}$ groups or together with the nitrogen atom form a 5 to 7-branched ring to which one or two further rings may be added, or

an acylaminomalonate group of formula

$$X\text{-}CO\text{-}HN\text{-}C(COO\text{-}alkyl\ C_{1-3})_2$$

wherein X has above denotation, a group of formula

$$X\text{-}CO\text{-}HN\text{-}\underset{|}{C}H\text{-}COOH$$

wherein X has above denotation, or a group of formula

$$H_2N\text{-}CH\text{-}\underset{|}{C}OOH;$$

and the salts thereof with mineral or organic acids.

**2.** Phenylalanine piperazides according to claim 1, wherein Y in the residue of formula —$SO_2$-Y is methyl, trifluoromethyl or trichloromethyl.

**3.** Phenylalanine piperazides according to claim 1 or 2, wherein $R^1$ represents an amidino group of formula (a) and/or $R^2$ means 2-naphthyl, anthraquinonyl, 2,4,6-triisopropylphenyl or 2,2,5,7,8-pentamethyl-chromanyl and/or $R^3$ represents an acyl residue of formula —COX, a functionalized alkyl residue of formula —$(CH_2)_n$-X', a residue of formula —$SO_2$-Y, a carboxamide residue of formula —$CONR^iR^{ii}$, 2-pyridyl or 2-pyrimidinyl.

**4.** Phenylalanine piperazides according to claim 3, wherein $R^3$ means formyl, acetyl or 2-hydroxyethyl.

**5.** Method for the synthesis of phenylalanine piperazides according to one of claims 1 — 4, wherein $R^1$ represents an amidino group of formula (a), which comprises converting a (D,L)-3-cyanophenylalanine alkylester of formula II

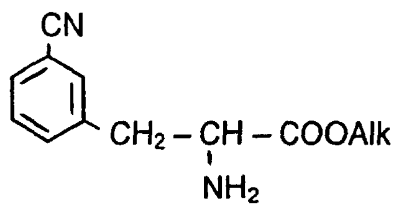

with a sulfochloride of formula $R^2$-$SO_2Cl$, wherein $R^2$ has the denotation given in claim 1, into a racemic compound of formula IV

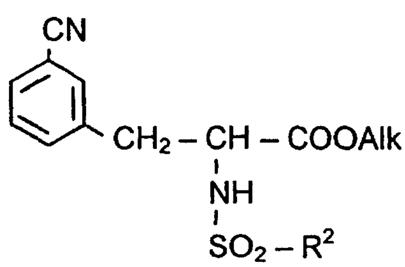

from which the racemate of the sulfonylated cyanophenylalanine of formula V

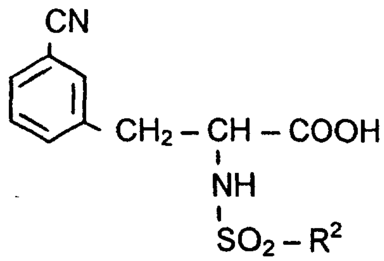

is obtained by hydrolysis, or converting the compound of formula IV by enzymatic ester hydrolysis with chymotrypsin into the L-configurated amino acid of formula V and converting the D-configurated amino alkyl carboxylates of formula IV obtained by this procedure into the D-configurated amino carboxylic acids of formula V by hydrolysis, converting the compound of formula V by coupling with a piperazine derivative of formula VII

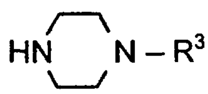

into the (D,L)-, D- or L-cyano compound with piperazide structure of formula VI

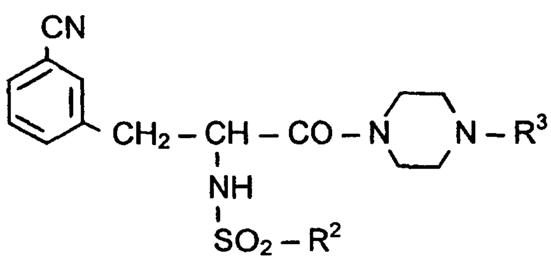

synthesizing the thioamide of formula VIII

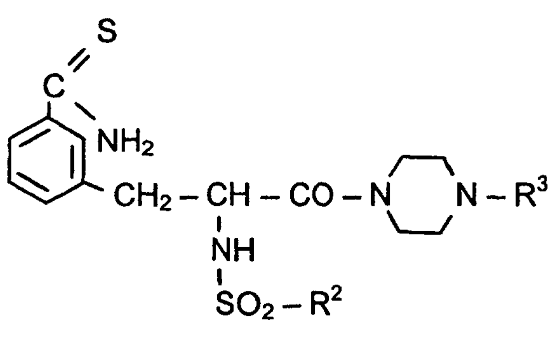

by addition of $H_2S$ to the cyano residue, synthesizing a thioimidate halide of formula IX

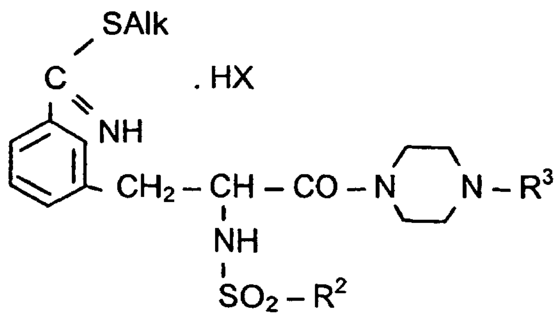

by conversion with an alkyl halide, or synthesizing an imidate halide of formula X

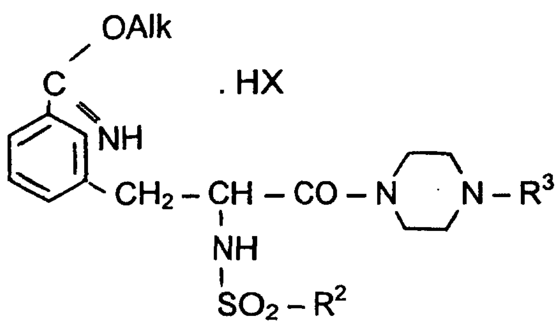

from the cyano compound with piperazide structure of formula VI, and converting the compound of formula IX with ammonium acetate or the compound of formula X in an alcoholic ammonia solution into a compound of formula XI

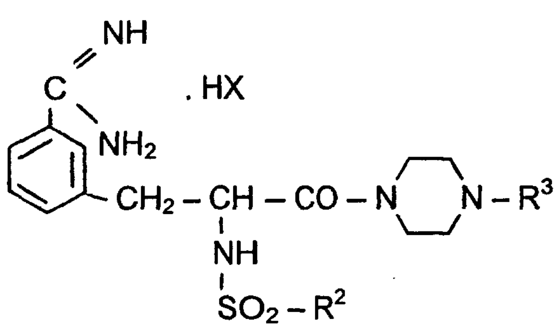

wherein X means halogen, preferentially chlorine.

**6.** Variant of the method according to claim 5 which, for the synthesis of (D,L)-, D- or L-cyano compound with piperazide structure of formula VI obtained as an intermediate, comprises protecting a (D,L), L- oder D-3-cyanophenylalanine by the introduction of a BOC group at the amino function, converting the obtained carboxylic acid of formula V, with the BOC group instead of the $SO_2$-$R^2$ group, with a piperazine derivative of formula VII into the corresponding BOC-protected (D,L)-, D- or L-cyano compound with piperazide structure of formula VI, with the BOC group instead of the $SO_2$-$R^2$ group, and converting this compound after acidic separation of the BOC group and conversion with a sulfochloride of formula $R^2$-$SO_2Cl$ into the compound of formula VI.

**7.** Method for the synthesis of phenylalanine piperazides according to one of claims 1 to 4, wherein $R^1$ means aminomethyl of formula (b), which comprises hydrogenating a cyano compound of formula VI as defined in claim 5.

**8.** Method according to claim 7 which comprises the catalytic hydrogenation of the compound of formula VI.

**9.** Use of the phenylalanine piperazides according to one of claims 1 to 4 as salts or free bases for the preparation of antithrombotically active drugs to be administered subcutaneously or intravenously, in particular orally, rectally or intraduodenally.

**10.** Antithrombotically active drug to be administered subcutaneously or intravenously, in particular however by the oral, rectal or duodenal route, in which an efficient quantity of at least one phenylalanine piperazide according to one of claims 1 to 4 and appropriate additives are present.

**11.** Antithrombotically active drug according to claim 10, in the form of tablets, dragées, capsules, pellets, suppositories, solutions, injections or transdermal systems, such as plasters.

**Revendications**

**1.** D,L-, L- ou D-phénylalanine pipérazides de formule

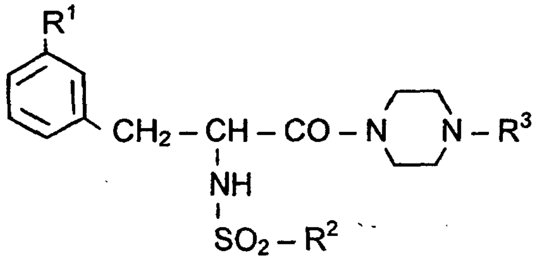

I

dans laquelle $R^1$ représente un groupement basique de formule

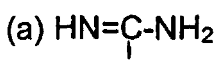

amidino

ou

(b) $-CH_2-NH_2$

aminométhyle

$R^2$ représente phényle, 4-méthylphényle, 2,4,6-triméthyl- ou — triisopropylphényle, 4-méthoxy-2,3,6-triméthylphényle, 2,2-diméthyl-6-méthoxy- ou 2,2,5,7,8-pentaméthylchromanyl, anthraquinonyl, 1- ou 2-naphtyl, quinolyl ou isoquinolyl ou un reste de camphre, respectivement; et

$R^3$ représente un reste acyle de formule —COX, où X signifie hydrogène, alkyle $C_{1-3}$ ou cycloalkyle $C_{3-10}$; ou

un reste benzyle où le reste aromatique peut être substitué avec un atome d'halogène ou un groupe alkyle $C_{1-3}$, alkoxy $C_{1-3}$, hydroxy ou nitro; ou

un reste carboxamide de formule —$CONR^iR^{ii}$, un reste thiocarboxamide de formule —$CSNR^iR^{ii}$ ou un reste éthylamide de formule —$CH_2$-$CONR^iR^{ii}$ où $R^i = R^{ii} = H$; $R^i = R^{ii}$ = alkyle $C_{1-3}$; $R^i = H$, $R^{ii}$ = alkyle $C_{1-3}$; $R^i = H$, $R^{ii}$ = phényle; ou $R^i$ et $R^{ii}$ forment avec l'atome d'azote un anneau de 5 à 7 branches pouvant contenir un hétéro atome supplémentaire; ou

un reste de formule —$SO_2$-Y où Y signifie alkyle $C_{1-3}$, trifluorométhyle, trichlorométhyle, phényle, 4-méthylphényle, 2,4,6-triméthyl- ou — triisopropylphényle, 4-méthoxy-2,3,6-triméthylphényle, 2,2-diméthyl-6-méthoxy- ou 2,2,5,7,8-pentaméthylchromanyl, anthraquinonyl, naphtyl, quinolyl ou —$NR^{iii}R^{iv}$, respectivement, où $R^{iii}$ et $R^{iv}$ = H et/ou égaux ou non à alkyle $C_{1-3}$; ou

un anneau cycloaliphatique $C_{5-8}$ pouvant être substitué avec un groupe hydroxyle ou oxo; ou

un reste pyridyle, pyrimidinyle ou N-méthylpipéridyle; ou

un reste alkyle fonctionnalisé de formule —$(CH_2)_n$-X' dans laquelle la chaîne alkyle est ramifiée ou non, n = 1 à 8 et le reste fonctionnel X' représente

un groupe hydroxy dont l'atome H peut être substitué avec de l'alkyle $C_{1-3}$, benzyle, phényle, 4-méthylphényle, 2,4,6-triméthyl- ou — triisopropylphényle, 4-méthoxy-2,3,6-triméthylphényle, anthraquinonyl, naphtyl,

hydroxy alkyle $C_{1-3}$ ou un groupe acyle de la formule — COX mentionnée ci-dessus, ou

un atome d'halogène, ou

un groupement aminé tertiaire de formule — $NR^vR^{vi}$ dans laquelle $R^v$ et $R^{vi}$ sont soit des groupes alkyle $C_{1-3}$ identiques, soit forment avec l'atome d'azote un anneau de 5 à 7 branches auquel on peut rajouter un ou deux anneaux supplémentaires, ou

un groupe acylaminomalonate de formule

$$X\text{-}CO\text{-}HN\text{-}C(COO\text{-alkyle } C_{1\text{-}3})_2$$

dans laquelle X a la signification mentionnée ci-dessus, un groupe de formule

$$X\text{-}CO\text{-}HN\text{-}CH\text{-}COOH$$

dans laquelle X a la signification mentionnée ci-dessus, ou un groupe de formule

$$H_2N\text{-}CH\text{-}COOH;$$

et leurs sels avec des acides minéraux ou organiques.

**2.** Phénylalanine pipérazides selon la revendication 1, où Y dans le reste de formule $-SO_2-Y$ représente méthyle, trifluorométhyle ou trichlorométhyle.

**3.** Phénylalanine pipérazides selon la revendication 1 ou 2, où $R^1$ représente un groupe amidino de formule (a) et/ou $R^2$ signifie 2-naphtyl, anthraquinonyl, 2,4,6-triisopropylphényle ou 2,2,5,7,8-pentaméthylchromanyl et/ou $R^3$ représente un reste acyle de formule — COX, un reste alkyle fonctionnalisé de formule — $(CH_2)_n$-X', un reste de formule $-SO_2-Y$, un reste carboxamide de formule — $CONR^iR^{ii}$, 2-pyridyle ou 2-pyrimidinyle.

**4.** Phénylalanine pipérazides selon la revendication 3, où $R^3$ représente un groupe formyle, acétyle ou 2-hydroxyéthyle.

**5.** Méthode de synthèse de phénylalanine pipérazides selon l'une des revendications 1 à 4, où $R^1$ représente un groupe amidino de formule (a), **caractérisée par** la conversion d'un alkylester de (D,L)-3-cyanophénylalanine de formule II

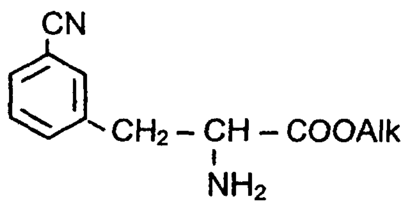

à l'aide d'un sulfochlorure de formule $R^2-SO_2Cl$, où $R^2$ a la signification mentionnée dans la revendication 1, en un composé racémique de formule IV

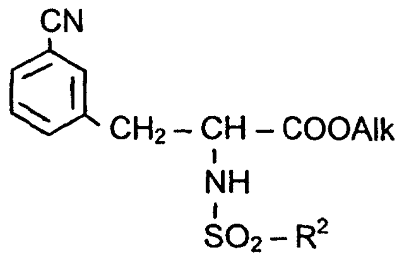

à partir duquel on obtient, par hydrolyse, le racémate de cyanophénylalanine sulfonylé de formule V

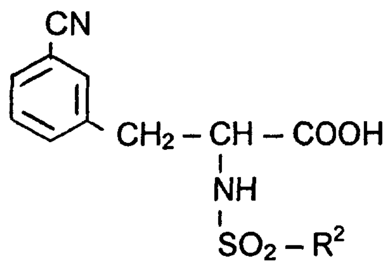

ou conversion du composé de formule IV par hydrolyse d'ester enzymatique avec de la chymotrypsine en un acide aminé de forme L et de formule V et conversion, par hydrolyse, des carboxylates d'alkyle aminés de forme D et de formule IV ainsi obtenus en acides aminés carboxyliques de forme D et de formule V, conversion du composé de formule V par couplage avec un dérivé de pipérazine de formule VII

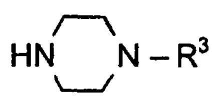

en un composé (D,L)-, D- ou L-cyano ayant une structure de pipérazide de formule VI

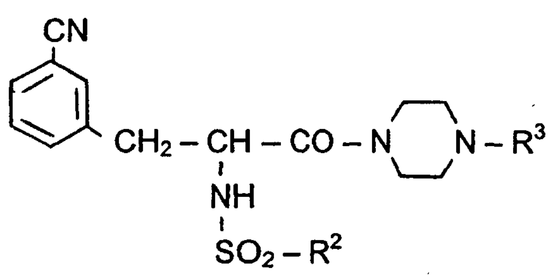

synthèse du thioamide de formule VIII

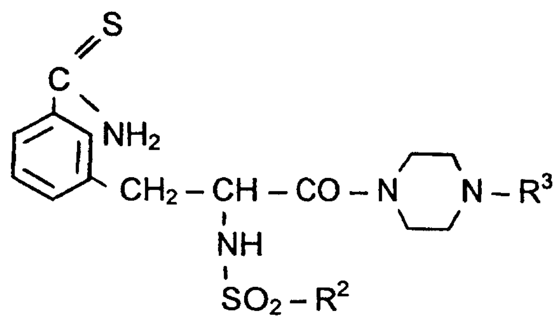

par addition de $H_2S$ au reste cyano, synthèse d'un halogénure de thioimidate de formule IX

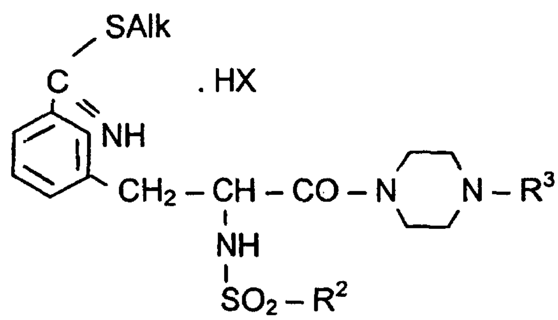

par conversion avec un halogénure d'alkyle, ou synthèse d'un halogénure d'imidate de formule X

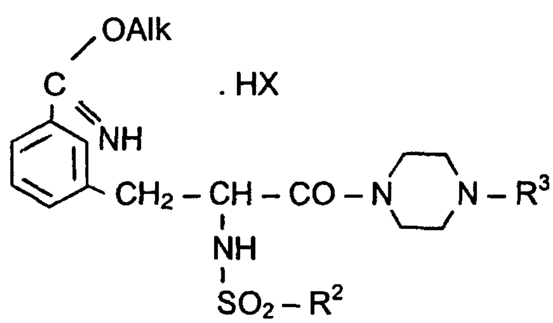

à partir du composé cyano ayant une structure de pipérazide de formule VI, et conversion du composé de formule IX avec de l'acétate d'ammonium ou du composé de formule X dans une solution alcoolique d'ammoniaque en un composé de formule XI

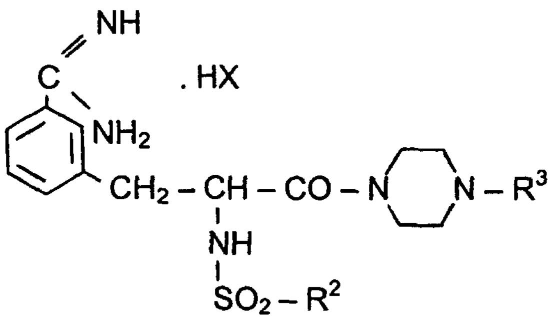

dans laquelle X représente de l'halogène, de préférence du chlore.

**6.** Variante de la méthode selon la revendication 5 qui, pour la synthèse du composé (D,L)-, D- ou L-cyano ayant une structure de pipérazide de formule VI obtenu comme intermédiaire, est **caractérisée par** la protection d'une (D,L), L- ou D-3-cyanophénylalanine par l'introduction d'un groupement BOC à la fonction amino, la conversion de l'acide carboxylique de formule V obtenu, toutefois avec un groupement BOC au lieu du groupement $SO_2$-$R^2$, avec un dérivé de pipérazine de formule VII en un composé (D,L)-, D- ou L-cyano BOC-protégé correspondant ayant une structure de pipérazide de formule VI, toutefois avec un groupement BOC au lieu du groupement $SO_2$-$R^2$, et la conversion de ce composé après séparation acide du groupement BOC et la conversion à l'aide d'un sulfochlorure de formule $R^2$-$SO_2Cl$ en un composé de formule VI.

**7.** Méthode de synthèse de phénylalanine pipérazides selon l'une des revendications 1 à 4, où $R^1$ signifie aminométhyle de formule (b), **caractérisée par** l'hydrogénation d'un composé cyano de formule VI comme défini dans la revendication 5.

**8.** Méthode selon la revendication 7, **caractérisée par** l'hydrogénation catalytique du composé de formule VI.

**9.** Utilisation des phénylalanine pipérazides selon l'une des revendications 1 à 4 sous forme de sels ou de bases libres pour la préparation de médicaments antithrombotiques à administrer par voie sous-cutanée ou intraveineuse, en particulier cependant par voie orale, rectale ou intraduodénale.

**10.** Médicament antithrombotique à administrer par voie sous-cutanée ou intraveineuse, en particulier cependant par voie orale, rectale ou duodénale, **caractérisé par** la présence d'une quantité efficiente d'au moins un phénylalanine pipérazide selon l'une des revendications 1 à 4 et d'additifs appropriés.

**11.** Médicament antithrombotique selon la revendication 10, sous forme de comprimés, de dragées, de capsules, de pilules, de suppositoires, de solutions, d'injections ou de systèmes transdermiques, tels que des emplâtres.